# EUROPEAN PATENT SPECIFICATION

(11) **EP 2 714 926 B1**
(45) Date of publication and mention of the grant of the patent: **22.07.2015**
(21) Application number: 12723210.6
(22) Date of filing: 24.05.2012
(51) Int. Cl.: C12Q 1/68

(54) **BIOMARKERS FOR LUNG CANCER**
BIOMARKER FÜR LUNGENKREBS
BIOMARQUEURS POUR LE CANCER DU POUMON

(30) Priority: 25.05.2011 US 201161490021 P
(43) Date of publication of application: 09.04.2014
(73) Proprietor: Novartis AG, 4056 Basel (CH)
(72) Inventor: MISSIAGLIA, Edoardo, 1015 Lausanne (CH); WIRAPATI, Pratyaksha, 1015 Lausanne (CH); ROSSI, Simona, 1015 Lausanne (CH); KROLL, Werner, Cambridge, MA 02139 (US)
(74) Representative: Kristl, Jernej
(86) International application number: PCT/EP2012/059784
(87) International publication number: WO 2012/160177

(56) References cited:
- WO-A1-2010/063121
- SUZANNE K LAU ET AL: "Three-gene prognostic classifier for early-stage non-small-cell lung cancer", JOURNAL OF CLINICAL ONCOLOGY, AMERICAN SOCIETY OF CLINICAL ONCOLOGY, US, vol. 25, no. 35, 10 December 2007 (2007-12-10), pages 5562-5569, XP008145645, ISSN: 0732-183X, DOI: 10.1200/JCO.2007.12.0352
- BOUTROS PAUL C ET AL: "Prognostic gene signatures for non-small-cell lung cancer.", PROCEEDINGS OF THE NATIONAL ACADEMY OF SCIENCES OF THE UNITED STATES OF AMERICA 24 FEB 2009 LNKD- PUBMED:19196983, vol. 106, no. 8, 24 February 2009 (2009-02-24), pages 2824-2828, XP002679415, ISSN: 1091-6490
- KADARA HUMAM ET AL: "Identification of Gene Signatures and Molecular Markers for Human Lung Cancer Prognosis using an In vitro Lung Carcinogenesis System", CANCER PREVENTION RESEARCH, vol. 2, no. 8, August 2009 (2009-08), pages 702-711 URL, XP002679416,
- PALLANTE P ET AL: "The loss of the CBX7 gene expression represents an adverse prognostic marker for survival of colon carcinoma patients", EUROPEAN JOURNAL OF CANCER, PERGAMON PRESS, OXFORD, GB, vol. 46, no. 12, 1 August 2010 (2010-08-01), pages 2304-2313, XP027189167, ISSN: 0959-8049 [retrieved on 2010-06-09]
- KARAMITOPOULOU EVA ET AL: "Loss of the CBX7 protein expression correlates with a more aggressive phenotype in pancreatic cancer", EUROPEAN JOURNAL OF CANCER, vol. 46, no. 8, May 2010 (2010-05), pages 1438-1444, XP002679417, ISSN: 0959-8049

## Description

### FIELD OF THE INVENTION

The present invention relates to a method of prognosis and personalized therapy.

### BACKGROUND OF THE INVENTION

Lung cancer is the most common cancer diagnosis in the world with 1.5 million new cases in 2007 (Salomon et al., Crit Rev Oncol Hematol., 19:183-232, 1995, SEER-database 05.2010). The high incidence and mortality rates make it the leading cause of cancer-related death with more than 975,000 deaths per year and a 5-year survival rate of 15% (Salomon et al., *supra*). Lung cancer can be classified as small cell lung cancer, or non-small cell lung cancer (NSCLC). NSCLC accounts for about 80% of the cases. NSCLC can be further subdivided into several histological types, the most common ones are adenocarcinoma (40%) and squamous cell carcinoma (25%).

The current treatment of NSCLC is mainly based on tumor morphology and the tumor-node-metastasis (TNM)-based staging system that classifies tumor in graduated categories (Stage IA, IB, IIA; IIB, IIIA, IIIB and IV) corresponding to the extent of tumor progression. Many staging systems exist (e.g., clinical vs. pathological staging, as well as various editions of staging guidelines such as those issued by International Association for the Study of Lung Cancer (IASLC) (Goldstraw et al, J. Thorac. Oncol. 2, 706-714,2007). The frequency of the stages, for example according to clinical staging and IASLC 6th edition of TNM staging recommendation, are 23% stage I, 19% stage II, 37% stage III and 21% stage IV (Goldstraw et al. *supra*). The relative proportions may change substantially depending on the guidelines and whether clinical or pathological staging is used.

Surgery is the standard treatment for early stage NSCLC (stage I and II), followed by adjuvant therapy such as radiation therapy, chemotherapy (for stage II and later), and bevacizumab and epidermal growth factor receptor (EGFR) tyrosine kinase inhibitors (TKIs) for the advanced NSCLC-stages (Kutikova et al., Lung Cancer; 50(2):143-154, 2005). Clinical guidelines in the United States and Europe for treatment of NSCLC support these treatment options (Mendelssohn et al., 2000; NCCN-Guidelines NSCLC V1, 2010).

Based on the current TNM-based staging system for early lung cancer Stage I NSCLC patients suffer from a 35 % chance of relapse within 5 years after surgery (SEER-Database, 2008). Current treatment guidelines do not recommend an adjuvant chemotherapy for these patients. Whereas 30 % patients with a TNM-based Stage II will not experience a relapse without any adjuvant chemotherapy (SEER-Database) meaning that these patients experience over treatment based on the current treatment guidelines (i.e., ESMO (D'Addario et al., Annals of Oncology. 2009; 20 (suppl 4):iv68-iv70, 2009), NCCN V1-2010). This is paralleled by reports stating that 60% of patients with early NSCLC will have no relapse after surgery (Arriagada et al., NEJM, 350:351-360, 2004). Based on current clinical data adjuvant chemotherapy treatment of early NSCLC provides evidence that the median benefit for adjuvant chemotherapy is 4 % (NSCLC Meta-Analysis Collaborative Group), improving from 60 % to 64 % at 5 years.

Based on the current shortcomings, there is a medical rational for the need of a prognostic and/or predictive genomic signature for patients with NSCLC.

### SUMMARY OF THE INVENTION

The present invention relates, in part, to methods for determining a prognosis of early stage lung cancer in an individual using one or more biomarkers described herein. These findings may be used to help to determine appropriate treatments for patients with early stage lung cancer such as identifying those patients who would benefit from receiving adjuvant therapy.

In one aspect, the invention includes a method for prognosing or classifying a subject with non-small cell lung cancer (NSCLC) including obtaining a test sample from a subject suffering from NSCLC following surgical resection; determining the expression level of at least one or more biomarker identified in Table 1, Table 2 and/or Table 3, or any combination of biomarkers identified in Table 1, Table 2 and/or Table 3 in the test sample; and analyzing the expression level to generate a risk score, wherein the risk score can be used to provide a prognosis or classify the subject.

In another aspect, the invention includes a method for prognosing or classifying a subject with non-small cell lung cancer (NSCLC) comprising: obtaining a test sample from a subject suffering from NSCLC following surgical resection; determining the expression level of at least one biomarkers from Table 1, Table 2 and Table 3 in the test sample; and analyzing the expression level to generate a risk score, wherein the risk score can be used to provide a prognosis or classify the subject. In one embodiment, the at least one biomarker identified in Table 1, Table 2 and Table 3 includes CBX7, STX1A, and TPX2. In another embodiment, the at least one biomarker identified in Table 1, Table 2 and Table 3 includes CBX7, TMPRSS2, STX1A, KLK6, TPX2 and UCK. In yet another embodiment, the at least one biomarker identified in Table 1, Table 2 and Table 3 includes CBX7, TMPRSS2, GPR116, STX1A, KLK6, SLC16A3, TPX2, UCK2, PHKA1. In still yet another embodiment, the at least one biomarker identified in Table 1, Table 2 and Table 3 comprises CBX7, TMPRSS2, GPR116, KCNJ15, STX1A, KLK6, SLC16A3, PYGL, TPX2, UCK2, PHKA1, or EIF4A3. In yet another embodiment, the at least one biomarker identified in Table 1, Table 2 and Table 3 includes CBX7, TMPRSS2, GPR116, KCNJ15, PTPN13, STX1A, KLK6, SLC16A3, PYGL, LDHA, TPX2, UCK2, PHKA1, EIF4A3 or TK1 . In yet another embodiment, the at least one biomarker identified in Table 1, Table 2 and Table 3 comprises CBX7, TMPRSS2, GPR116, KCNJ15, PTPN13, CTSH, STX1A, KLK6, SLC16A3, PYGL, LDHA, ITGA5, TPX2, UCK2, PHKA1, EIF4A3, TK1, or CCNA2.

In one embodiment, the risk score of the invention can be used for prognosis by mapping subjects to time-specific probability of death due to lung cancer, distance metastasis or local relapse.

In another embodiment, the risk score can classify the subject into a high risk group that would benefit from receiving adjuvant chemotherapy or in a low risk group that would not benefit from receiving adjuvant chemotherapy.

In another aspect, the invention includes a method of predicting prognosis in a subject with non-small cell lung cancer (NSCLC) following surgical resection, comprising determining expression profile of mRNA from tumor samples, either from fresh frozen (FF) or formalin fixed paraffin embedded (FFPE) material. The profile comprises of one or more biomarkers listed in Table 1, Table 2 and/or Table 3, wherein an increase in expression of one or more biomarkers listed in Table 2 and/or Table 3 and a decrease in expression of one or more of the biomarkers listed in Table 1 compared to a control is used to predict whether the subject is in a high risk group having poor survival or a low risk group having good survival. In the method of the invention, a subject in the high risk group is selected for adjuvant chemotherapy and the subject in the low risk group is not selected for adjuvant chemotherapy and then treated accordingly.

In yet another embodiment, the invention includes a method of selecting a therapy for a subject with NSCLC, including obtaining a test sample from a subject suffering from NSCLC who has undergone a resection; determining the expression level of at least two or more biomarkers identified in Table 2 in the test sample to generate an expression value for each gene; and analyzing the expression value to generate a risk score, wherein the risk score can be used to classify whether the subject is selected to receive an angiogenesis inhibitor such as avastin.

In the methods of the invention, the invention includes determining expression of at least 2, at least 3, at least 4, at least 5, at least 6, at least 7, at least 8, at least 9, at least10, at least 11, at least 12, at least 13, at least 14 or at least 15 biomarkers identified in Table 1, Table 2 and/or Table 3, or any combination thereof. For example, the expression of at least any 5 biomarkers from each of Table 1, Table 2 and Table 3 are selected (the signature in this embodiment would include at least 15 biomarkers).

In one embodiment, the NSCLC is stage I NSCLC, stage II NSCLC, or a combination thereof. The NSCLC can be identified in the group consisting of squamous cell carcinoma and/or adenocarcinoma.

In one embodiment, the subject is human. In another embodiment, the test sample can be fresh, frozen, FFPE cells. In another embodiment, the expression level is determined using quantitative PCR or an array.

In yet another embodiment, analyzing expression to generate a risk score is performed using statistical analysis such as Cox regression or parametric survival predictors.

In another aspect, the invention includes a method of selectively treating a subject having NSCLC cancer including obtaining a test sample from a subject suffering from NSCLC following surgical resection, determining the expression level of at least one or more biomarkers identified in Table 1, Table 2 and/or Table 3, or any combination of biomarkers identified in Table 1, Table 2 and/or Table 3 in the test sample to generate a risk score; classifying the subject based on the risk score into a high risk group or a low risk group; and administering adjuvant therapy to the subject classified as belonging to the high risk group or administering no adjuvant therapy to the subject classified as belonging to the low risk group.

In yet another aspect, the invention includes a kit including a plurality of agents for measuring the expression of one or more biomarkers identified in Table 1, Table 2 and/or Table 3 and instructions for use. In yet another aspect, the invention includes a kit for predicting whether a subject with lung cancer would benefit from adjuvant therapy, the kit includes a plurality of agents for measuring the expression of one or more biomarkers identified in Table 1, Table 2 and/or Table 3; means for analyzing the expression and generating a risk score to predict whether a patient would benefit from adjuvant therapy. The agents for measuring expression can include an array of polynucleotides complementary to the mRNAs of the identified biomarkers. The agents that measure expression can include a plurality of PCR probes and/or primers for qRT-PCR. The kit can include agents for measuring at least one biomarker identified in Table 1, Table 2 and Table 3 such as CBX7, STX1A, or TPX2.

In another aspect, the invention includes an array comprising one or more polynucleotide probes complementary and hybrdizable to an expression product of at least two biomarkers etc shown in Table 1, Table 2 and/or Table 3.

In yet another aspect, the invention includes a composition comprising a plurality of isolated nucleic acid sequences, wherein each isolated nucleic acid sequence hybridizes to an RNA product of a biomarker shown in Table 1, e.g., the biomarkers CBX7, STX1A and TPX2, wherein the composition is used to measure the level of RNA expression of the three genes.

In yet another aspect, the invention includes a computer product for predicting a prognosis, or classifying a subject with NSCLC including means for receiving data corresponding to the expression level of one or more biomarkers in a sample from a subject having NSCLC, wherein the one or more biomarkers are identified in Table 1, Table 2 and/or Table 3, means for generating an expression value for each gene; and means for generating a risk score based on inputting the expression value into a database comprising a reference expression profile associated with a prognosis, wherein the risk score predicts a prognosis of survival or classifies the subject into a high risk group or a low risk group.

In yet another aspect, the invention includes a computer product for use with the method of any one of methods described above.

A "biomarker" is a molecule useful as an indicator of a biologic state in a subject. With reference to the present subject matter, the biomarkers disclosed herein can be molecules that exhibit a change in expression and whose presence can be used for prognosis or to predict whether a subject would benefit from receiving a particular treatment. The biomarkers of interest can be determined by detecting for a change in expression of the biomarker. A change in expression describes the conversion of the DNA gene sequence information into transcribed RNA (the initial unspliced RNA transcript or the mature mRNA) or the encoded protein product. The biomarkers disclosed herein include any, or any combination of the biomarkers listed in Tables 1, 2 and 3 and can be transcribed RNA or encoded protein product.

### BRIEF DESCRIPTION OF THE DRAWINGS

**Fig. 1** depicts pairwise scatter plots of the standardized scores of modules 1, 2 and 3, demonstrating they convey different information about the tumor.
**Fig 2A-D** depict a Kaplan-Meier overall survival analysis curves showing the prognostic performance of the three signatures (module 1,2, and 3) when they are applied separately, or combined, for patients up to five years after surgery.
**Fig. 3A-F** depict a Kaplan-Meier overall survival analysis curves showing the prognostic performance of module 1 under stratification by stage, histology and age, for patients up to five years after surgery.
**Fig. 4A-F** depict a Kaplan-Meier survival analysis curves showing the prognostic performance of module module 2 under stratification by stage, histology and age, for patients up to five years after surgery.
**Fig. 5A-F** depict a Kaplan-Meier survival analysis curves showing the prognostic performance of module 3 under stratification by stage, histology and age, for patients up to five years after surgery.
**Fig. 6A-F** depict a Kaplan-Meier survival analysis curves showing the prognostic performance of combined score under stratification by stage, histology and age, for patients up to five years after surgery.
**Fig. 7A-F** depict Kaplan-Meier survival analysis curves showing the prognostic performance of a number of example signatures constructed as subsets of the 38-gene list.
**Fig. 8A** depicts the lung cancer percentage mortality (equivalent to 100% minus survival) as the function of the risk score of the claimed signature and shows such prognosis at 1-, 2-, 3-, 4- and 5-year follow-up time. **Fig. 8B** depicts the lung cancer percentage mortality (equivalent to 100% minus survival) as the function of the risk score of the claimed signature and shows the 5-year mortality as the function of risk score, stratified by tumor stage I and 2.
**Fig. 9** depicts a Kaplan-Meier survival analysis curve showing the survival performance in publicly available dataset from M. D. Anderson Cancer Center which were obtained from FFPE material.
**Fig. 10A-B** depict pairwise scatter plots comparing FF/Affymetrix vs FFPE/qNPA, as well as FF/Affymetrix vs FFPE/nanoString.

### DETAILED DESCRIPTION OF THE INVENTION

The present invention is based, in part, on methods which can be used for the prognosis or classification of individuals having early stage lung cancer. The invention further includes identifying those patients who are at high risk for disease recurrence and for whom adjuvant therapy might be recommended, as well as patients with a low recurrence risk, who might not benefit from adjuvant therapy. In one example, the prognosis and prediction methods described herein are based upon the differential expression of a plurality of biomarkers in a lung cancer test sample. The biomarkers of the invention can include 38 genes (CBX7, TMPRSS2, GPR116, KCNJ15, PTPN13, CTSH, PPFIBP2, CD302, SFTPB, HSD17B6, DLC1, ADRB2, PARM1, KLRB1, MS4A1, STX1A, KLK6, SLC16A3, PYGL, LDHA, ITGA5, VEGFC, EEF1A2, TPX2, UCK2, PHKA1, EIF4A3, TK1, CCNA2, GGH, CCNB1, MELK, HMMR, EIF2S1, TEAD4, HMGA1, RIMS2, H2AFZ), or a combination thereof, which can be broken up into three modules (Table 1,2, and 3) based on criteria including biological function. Table 1 (which is referred to herein as also Module 1) includes genes involved in tumor suppression, Table 2 (which is referred to herein as also Module 2) includes genes involved in angiogenesis, and Table 3 (which is referred to herein as also Module 3) includes genes involved in proliferation.

It was discovered that some biomarkers are over-expressed in early stage lung cancer such as those markers involved in angiogensis or proliferation (Table 2 and Table 3, respectively), whereas other biomarkers involved in tumor suppression are under-expressed (Table 1) as compared to a control (e.g., the average expression of these genes in patients with early stage lung cancer (stage I and II)).

### Biomarker

The biomarker(s) of the invention includes one or more biomarkers listed in Table 1, Table 2, and/or Table 3, or their gene products. The present invention is based on the finding that the biomarkers listed in Table 1, Table 2, and/or Table 3 are differentially expressed. By analyzing the expression profile levels of one or more biomarkers identified in Table 1, Table 2, and/or Table 3 it is possible to determine the prognosis of an individual with early stage lung cancer.

In one example, the method of the invention includes measuring one or more biomarkers from Table 1. For example, the method of the invention measures at least one, at least two, at least three, at least four, at least five, at least six, at least seven, at least eight, at least nine, at least ten, at least eleven, at least twelve, at least thirteen, at least fourteen or at least fifteen, biomarkers from Table 1. In one example, the level of expression of one gene CBX7 from Table 1 is measured. In another example, the level of expression of two biomarkers CBX7 and TMPRSS2 from Table 1 are measured. In yet another example, the level of expression of three biomarkers CBX7, TMPRSS2 and GPR116 from Table 1 are measured. In yet another example, the level of expression of four biomarkers CBX7, TMPRSS2, GPR116 and KCNJ15 from Table 1 are measured. In yet another example, the level of expression of five biomarkers CBX7, TMPRSS2, GPR116, KCNJ15 and PTPN13 from Table 1 are measured.

**Table 1**

| **Gene symbol** | **Entrez ID** | **Description** | **Accession** | **Symbol Alias** |
|---|---|---|---|---|
| CBX7 | 23492 | chromobox homolog 7 | NM_175709.3 | |
| TMPRSS2 | 7113 | transmembrane protease, serine 2 | NM_005656.3; NM_001135099.1 | FLJ41954; PP9284; PRSS10 |
| GPR116 | 221395 | G protein-coupled receptor 116 | NM_015234.4; NM_001098518.1 | DKFZp564O192 FLJ90640; KIAA0758; KPG 001 |
| KCNJ15 | 3772 | potassium inwardly-rectifying channel, subfamily J, member 15 | NM_170736.1; NM_170737.1; NM_002243.3 | IRKK; KIR1.3; KIR4.2; MGC13584 |
| PTPN13 | 5783 | protein tyrosine phosphatase, non-receptor type 13 (APO-1/CD95 (Fas)-associated phosphatase) | NM_080684.2; NM_006264.2; NM_080685.2; NM_080683.2 | DKFZp686J1497; FAP-1; PNP1; PTP-BAS; PTP-BL; PTP1E; PTPL1; PTPLE |
| CTSH | 1512 | cathepsin H | NM_004390.3 | ACC-4; ACC-5; CPSB; DKFZp686B24257; MGC1519; minichain |
| PPFIBP2 | 8495 | PTPRF interacting protein, binding protein 2 (liprin beta 2) | NM_003621.2 | Cclp1; DKFZp781K06126; MGC42541 |
| CD302 | 9936 | CD302 molecule | NM_001198763.1; NM_001198764.1; NM_014880.4 | BIMLEC; CLEC13A; DCL-1; DCL1; FLJ43091; KIAA0022; MGC22301 |
| SFTPB | 6439 | surfactant protein B | NM_198843.2; NM_000542.3 | PSP-B; SFTB3; SFTP3; SMDP1; SP-B |
| HSD17B6 | 8630 | hydroxysteroid (17-beta) dehydrogenase 6 homolog (mouse) | NM_003725.2 | HSE; RODH; SDR9C6 |
| DLC1 | 10395 | deleted in liver cancer 1 | NM_182643.2; NM_024767.3; NM_006094.4; NM_001164271.1 | ARHGAP7; FLJ21120; HP; p122-RhoGAP; STARD12 |
| ADRB2 | 154 | adrenergic, beta-2-, receptor, surface | NM_000024.5 | ADRB2R; ADRBR; B2AR; BAR;BETA2AR |
| PARM1 | 25849 | prostate androgen-regulated mucin-like protein 1 | NM_015393.3 | Cipar1; DKFZP564O0823; PARM-1; WSC4 |
| KLRB1 | 3820 | killer cell lectin-like receptor subfamily B, member 1 | NM_002258.2 | CD161; CLEC5B; hNKR-P1A; MGC138614; NKR; NKR-P1; NKR-P1A; NKRP1A |
| MS4A1 | 931 | membrane-spanning 4-domains, subfamily A, member 1 | NM_152866.2; NM_021950.3 | B1; Bp35; CD20; CVID5; LEU-16; MGC3969; MS4A2; S7 |

In another example, the method of the invention includes measuring one or more biomarkers from Table 2. For example, the method of the invention measures the expression of at least one, at least two, at least three, at least four, at least five, at least six, at least seven or at least eight biomarkers from Table 2. In one example, the level of expression of one gene STX1A from Table 2 is measured. In one example, the level of expression of two biomarkers STX1A and KLK6 from Table 2 are measured. In another example, the level of expression of three biomarkers STX1A, KLK6 and SLC16A3 from Table 2 are measured. In another example, the level of expression of four biomarkers STX1A, KLK6, SLC16A3 and PYGL from Table 2 are measured. In yet another example, the level of expression of five biomarkers STX1A, KLK6, SLC16A3, PYGL and LDHA from Table 2 are measured.

**Table 2**

| **Gene symbol** | **Entrez ID** | **Description** | **Accession** | **Symbol Alias** |
|---|---|---|---|---|
| STX1A | 6804 | syntaxin 1A (brain) | NM_004603.3; NM_001165903.1 | HPC-1; P35-1; STX1; SYN1A |
| KLK6 | 5653 | kallikrein-related peptidase 6 | NM_002774.3; NM_001012964.1; NM_001012965.1 | Bssp; hK6; Klk7; MGC9355; PRSS18; PRSS9; SP59 |
| SLC16A3 | 9123 | solute carrier family 16, member 3 (monocarboxylic acid transporter 4) | NM_001042422.1; NM_001042423.1; NM_004207.2 | MCT3; MCT4; MGC138472; MGC138474 |
| PYGL | 5836 | phosphorylase, glycogen, liver | NM_002863.4; NM_001163940.1 | GSD6 |
| LDHA | 3939 | lactate dehydrogenase A | NR_028500.1; NM_001165415.1; NM_005566.3; NM_001165416.1; NM_001135239.1; NM_001165414.1 | GSD11; LDH1; LDHM; PIG19 |
| ITGA5 | 3678 | integrin, alpha 5 (fibronectin receptor, alpha polypeptide) | NM_002205.2 | CD49e; FNRA; VLA5A |
| VEGFC | 7424 | vascular endothelial growth factor C | NM_005429.2 | Flt4-L; VRP |
| EEF1A2 | 1917 | eukaryotic translation elongation factor 1 alpha 2 | NM_001958.2 | EEF1AL; EF-1-alpha-2; EF1A; FLJ41696; HS1; STN; STNL |

In another example, the method of the invention includes measuring one or more biomarkers from Table 3. For example, the method of the invention measures at least one, at least two, at least three, at least four, at least five, at least six, at least seven, at least eight, at least nine, at least ten, at least eleven, at least twelve, at least thirteen, at least fourteen or at least fifteen, biomarkers from Table 3. In one example, the level of expression of one gene TPX2 from Table 3 is measured. In another example, the level of expression of two biomarkers TPX2 and UCK2 from Table 3 are measured. In another example, the level of expression of three biomarkers TPX2, UCK2 and PHKA1 from Table 3 are measured. In another example, the level of expression of four biomarkers TPX2, UCK2, PHKA1 and EIF4A3 from Table 3 are measured. In yet another example, the level of expression of five biomarkers TPX2, UCK2, PHKA1 EIF4A3 and TK1 from Table 3 are measured.

**Table 3**

| **Gene symbol** | **EntrezID** | **Description** | **Accession** | **Symbol Alias** |
|---|---|---|---|---|
| TPX2 | 22974 | TPX2, microtubule-associated, homolog (Xenopus laevis) | NM_012112.4 | C20orf1; C20orf2; DIL-2; DIL2; FLS353; GD:C20orf1; HCA519; HCTP4; p100; REPP86 |
| UCK2 | 7371 | uridine-cytidine kinase 2 | NM_012474.4 | DKFZp686M04245; TSA903; UK; UMPK |
| PHKA1 | 5255 | phosphorylase kinase, alpha 1 (muscle) | NM_001172436.1; NM_002637.3; NM_001122670.1 | MGC132604; PHKA |
| EIF4A3 | 9775 | eukaryotic translation initiation factor 4A3 | NM_014740.3 | DDX48; DKFZp686O16189; eIF4AIII; KIAA0111; MGC10862; NMP265; NUK34 |
| TK1 | 7083 | thymidine kinase 1, soluble | NM_003258.4 | TK2 |
| CCNA2 | 890 | cyclinA2 | NM_001237.3 | CCN1; CCNA |
| GGH | 8836 | gamma-glutamyl hydrolase (conjugase, folylpolygammaglutamyl hydrolase) | NM_003878.2 | GH |
| CCNB1 | 891 | cyclin B1 | NM_031966.2 | CCNB |
| MELK | 9833 | maternal embryonic leucine zipper kinase | NM_014791.2 | HPK38; KIAA0175 |
| HMMR | 3161 | hyaluronan-mediated motility receptor (RHAMM) | NM_012485.2; NM_001142557.1; NM_001142556.1; NM_012484.2 | CD168; IHABP; MGC119494; MGC119495; RHAMM |
| EIF2S1 | 1965 | eukaryotic translation initiation factor 2, subunit 1 alpha, 35kDa | NM_004094.4 | EIF-2; EIF-2A; EIF-2alpha; EIF2; EIF2A |
| TEAD4 | 7004 | TEA domain family member 4 | NM_003213.3; NM_201441.2; NM_201443.2 | EFTR-2; hRTEF-1B; MGC9014; RTEF1; TCF13L1; TEF-3; TEF3; TEFR-1 |
| HMGA1 | 3159 | high mobility group AT-hook 1 | NM_145903.2; NM_002131.3; NM_145899.2; NM_145902.2; NM_145901.2; NM_145905.2 | HMG-R; HMGA1A; HMGIY; MGC12816; MGC4242; MGC4854 |
| RIMS2 | 9699 | regulating synaptic membrane exocytosis 2 | NM_001100117.2; NM_014677.4 | DKFZp781A0653; KIAA0751; OBOE; RAB3IP3; RIM2 |
| H2AFZ | 3015 | H2A histone family, member Z | NM_002106.3 | H2A.z; H2A/z; H2AZ; MGC117173 |

The biomarkers of the invention can also include any combination of biomarkers identified in Table 1, Table 2 and Table 3 whose level of expression or gene product serves as a predictive marker or biomarker for prognosis of an individual with early stage lung cancer. In one example, the level of expression of one gene selected from each Table, Table 1, Table 2 and Table 3 is measured, e.g., CBX7, STX1A and TPX2. In another example, the level of expression of two biomarkers selected from each of the Tables, Table 1, Table 2 and Table 3 is measured, e.g., CBX7 and TMPRSS2 from Table 1, STX1A and KLK6 from Table 2 and TPX2 and UCK2 from Table 3. See Table 4 below for examples of various combinations of biomarkers from Tables 1, 2 and 3. The combinations shown in Table 4 are not meant to be construed as limiting and any combination of biomarkers shown in Tables 1-3 can be made.

**Table 4**

| **Biomarker and Biomarker Combination** | **p-value** |
|---|---|
| CBX7 | 4.90E-06 |
| CBX7 TMPRSS2 | 1.10E-07 |
| CBX7 TMPRSS2 GPR116 | 1.90E-07 |
| CBX7 TMPRSS2 GPR116 KCNJ15 | 1.10E-08 |
| CBX7 TMPRSS2 GPR116 KCNJ15 PTPN13 | 5.50E-09 |
| CBX7 TMPRSS2 GPR116 KCNJ15 PTPN13 CTSH | 2.60E-09 |
| CBX7 TMPRSS2 GPR116 KCNJ15 PTPN13 CTSH PPFIBP2 | 3.60E-10 |
| CBX7 TMPRSS2 GPR116 KCNJ15 PTPN13 CTSH PPFIBP22 CD302 | 2.10E-10 |
| CBX7 TMPRSS2 GPR116 KCNJ15 PTPN13 CTSH PPFIBP2 CD302 SFTPB | 1.10E-10 |
| CBX7 TMPRSS2 GPR116 KCNJ15 PTPN13 CTSH PPFIBP2 CD302 SFTPB HSD17B6 | 1.90E-10 |
| CBX7 TMPRSS2 GPR116 KCNJ15 PTPN13 CTSH PPFIBP2 CD302 SFTPB HSD17B6 DLC1 | 1.60E-10 |
| CBX7 TMPRSS2 GPR116 KCNJ15 PTPN13 CTSH PPFIBP2 CD302 SFTPB HSD17B6 DLC1 ADRB2 | 8.50E-11 |
| CBX7 TMPRSS2 GPR116 KCNJ15 PTPN13 CTSH PPFIBP2 CD302 SFTPB HSD17B6 DLC1 ADRB2 PARM1 | 8.40E-11 |
| CBX7 TMPRSS2 GPR116 KCNJ15 PTPN13 CTSH PPFIBP2 CD302 SFTPB HSD17B6 DLC1 ADRB2 PARM1 KLRB1 | 3.60E-11 |
| CBX7 TMPRSS2 GPR116 KCNJ15 PTPN13 CTSH PPFIBP2 CD302 SFTPB HSD17B6 DLC1 ADRB2 PARM1 KLRB1 MS4A1 | 5.90E-12 |
| STX1A | 3.20E-04 |
| STX1A KLK6 | 1.40E-05 |
| STX1A KLK6 SLC16A3 | 1.50E-06 |
| STX1A KLK6 SLC16A3 PYGL | 6.90E-08 |
| STX1A KLK6 SLC16A3 PYGL LDHA | 2.10E-08 |
| STX1A KLK6 SLC16A3 PYGL LDHA ITGA5 | 3.00E-08 |
| STX1A KLK6 SLC16A3 PYGL LDHA ITGA5 VEGFC | 1.40E-08 |
| STX1A KLK6 SLC16A3 PYGL LDHA ITGA5 VEGFC EEF1A2 | 6.90E-08 |
| TPX2 | 2.20E-05 |
| TPX2 UCK2 | 4.30E-07 |
| TPX2 UCK2 PHKA1 | 5.40E-08 |
| TPX2 UCK2 PHKA1 EIF4A3 | 1.30E-08 |
| TPX2 UCK2 PHKA1 EIF4A3 TK1 | 2.60E-08 |
| TPX2 UCK2 PHKA1 EIF4A3 TK1 CCNA2 | 6.60E-08 |
| TPX2 UCK2 PHKA1 EIF4A3 TK1 CCNA2 GGH | 4.40E-08 |
| TPX2 UCK2 PHKA1 EIF4A3 TK1 CCNA2 GGH CCNB1 | 1.50E-07 |
| TPX2 UCK2 PHKA EIF4A3 TK1 CCNA2 GGH CCNB1 MELK | 2.60E-07 |
| TPX2 UCK2 PHKA1 EIF4A3 TK1 CCNA2 GGH CCNB1 MELK HMMR | 2.80E-07 |
| TPX2 UCK2 PHKA1 EIF4A3 TK1 CCNA2 GGH CCNB1 MELK HMMR EIF2S1 | 1.70E-07 |
| TPX2 UCK2 PHKA1 EIF4A3 TK1 CCNA2 GGH CCNB1 MELK HMMR EIF2S1 TEAD4 | 1.20E-07 |
| TPX2 UCK2 PHKA EIF4A3 TK1 CCNA2 GGH CCNB1 MELK HMMR EIF2S1 TEAD4 HMGA1 | 1.60E-07 |
| TPX2 UCK2 PHKA1 EIF4A3 TK1 CCNA2 GGH CCNB1 MELK HMMR EIF2S1 TEAD4 HMGA1 RIMS2 | 7.60E-08 |
| TPX2 UCK2 PHKA1 EIF4A3 TK1 CCNA2 GGH CCNB1 MELK HMMR EIF2S1 TEAD4 HMGA1 RIMS2 H2AFZ | 7.00E-08 |
| CBX7 | 3.10E-09 |
| STX1A | |
| TPX2 | |
| CBX7 TMPRSS2 | 1.60E-10 |
| STX1A KLK6 | |
| TPX2 UCK2 | |
| CBX7 TMPRSS2 GPR116 | 6.10E-11 |
| STX1A KLK6 SLC16A3 | |
| TPX2 UCK2 PHKA1 | |
| CBX7 TMPRSS2 GPR116 KCNJ15 | 1.90E-12 |
| STX1A KLK6 SLC16A3 PYGL | |
| TPX2 UCK2 PHKA1 EIF4A3 | |
| CBX7 TMPRSS2 GPR116 KCNJ15 PTPN13 | 1.80E-12 |
| STX1A KLK6 SLC16A3 PYGL LDHA | |
| TPX2 UCK2 PHKA1 EIF4A3 TK1 | |
| CBX7 TMPRSS2 GPR116 KCNJ15 PTPN13 CTSH | 3.20E-12 |
| STX1A KLK6 SLC16A3 PYGL LDHA ITGA5 | |
| TPX2 UCK2 PHKA1 EIF4A3 TK1 CCNA2 | |
| CBX7 TMPRSS2 GPR116 KCNJ15 PTPN13 CTSH PPFIBP2 | 1.40E-12 |
| STX1A KLK6 SLC16A3 PYGL LDHA ITGA5 VEGFC | |
| TPX2 UCK2 PHKA1 EIF4A3 TK1 CCNA2 GGH | |
| CBX7 TMPRSS2 GPR116 KCNJ15 PTPN13 CTSH PPFIBP2 CD302 | 3.10E-12 |
| STX1A KLK6 SLC16A3 PYGL LDHA ITGA5 VEGFC EEF1A2 | |
| TPX2 UCK2 PHKA1 E1F4A3 TK1 CCNA2 GGH CCNB1 | |
| CBX7 TMPRSS2 GPR116 KCNJ15 PTPN13 CTSH PPFIBP2 CD302 SFTPB | 2.80E-12 |
| STX1A KLK6 SLC16A3 PYGL LDHA ITGA5 VEGFC EEF1A2 | |
| TPX2 UCK2 PHKA1 EIF4A3 TK1 CCNA2 GGH CCNB1 MELK | |
| CBX7 TMPRSS2 GPR116 KCNJ15 PTPN13 CTSH PPFIBP2 CD302 SFTPB HSD17B6 | 4.00E-12 |
| STX1A KLK6 SLC16A3 PYGL LDHA ITGA5 VEGFC EEF1A2 | |
| TPX2 UCK2 PHKA1 EIF4A3 TK1 CCNA2 GGH CCNB1 MELK HMMR | |
| CBX7 TMPRSS2 GPR116 KCNJ15 PTPN13 CTSH PPFIBP2 CD302 SFTPB HSD17B6 DLC1 | 3.50E-12 |
| STX1A KLK6 SLC16A3 PYGL LDHA ITGA5 VEGFC EEF1A2 | |
| TPX2 UCK2 PHKA1 EIF4A3 TK1 CCNA2 GGH CCNB1 MELK HMMR EIF2S1 | |
| CBX7 TMPRSS2 GPR116 KCNJ15 PTPN13 CTSH PPFIBP2 CD302 SFTPB HSD17B6 DLC1 ADRB2 | 2.60E-12 |
| STX1A KLK6 SLC16A3 PYGL LDHA ITGA5 VEGFC EEF1A2 | |
| TPX2 UCK2 PHKA1 EIF4A3 TK1 CCNA2 GGH CCNB1 MELK HMMR EIF2S1 TEAD4 | |
| CBX7 TMPRSS2 GPR116 KCNJ15 PTPN13 CTSH PPFIBP2 CD302 SFTPB | 3.50E-12 |
| HSD17B6 DLC1 ADRB2 PARM1 | |
| STX1A KLK6 SLC16A3 PYGL LDHA ITGA5 VEGFC EEF1A2 | |
| TPX2 UCK2 PHKA1 EIF4A3 TK1 CCNA2 GGH CCNB1 MELK HMMR EIF2S1 | |
| TEAD4 HMGA1 | |
| CBX7 TMPRSS2 GPR116 KCNJ15 PTPN13 CTSH PPFIBP2 CD302 SFTPB | 1.90E-12 |
| HSD17B6DLC1 ADRB2 PARM1 KLRB1 | |
| STX1A KLK6 SLC16A3 PYGL LDHA ITGA5 VEGFC EEF1A2 | |
| TPX2 UCK2 PHKA1 EIF4A3 TK1 CCNA2 GGH CCNB1 MELK HMMR EIF2S1 | |
| TEAD4 HMGA1 RIMS2 | |
| CBX7 TMPRSS2 GPR116 KCNJ15 PTPN13 CTSH PPFIBP2 CD302 SFTPB | 9.60E-13 |
| HSD17B6 DLC1 ADRB2 PARM1 KLRB1 MS4A1 | |
| STX1A KLK6 SLC16A3 PYGL LDHA ITGA5 VEGFC EEF1A2 | |
| TPX2 UCK2 PHKA1 EIF4A3 TK1 CCNA2 GGH CCNB1 MELK HMMR EIF2S1 | |
| TEAD4 HMGA1 RIMS2 H2AFZ | |

In another example, at least any 3, 4, 5, 6, 7, 8, 9, 10 genes from each Table 1, Table 2, and Table 3 are selected. For example, in one embodiment, at least 15 biomarkers are selected where any 5 biomarkers from Table 1 are selected (e.g., CBX7, TMPRSS2, GPR116, KCNJ15 and PTPN13 or CBX7, TMPRSS2, CTSH, PPFIBP2 and CD302; or SFTPB; HSD17B6; DLC1; ADRB2 and PARM1), any 5 biomarkers from Table 2 are selected (e.g., STX1A, KLK6, SLC16A3, PYGL and LDHA or SLC16A3, PYGL, ITGA5, VEGFC, and EEF1A2 or STX1A, KLK6, SLC16A3, PYGL and LDHA) and any 5 biomarkers from Table 3 are selected (TPX2, UCK2, PHKA1, EIF4A3, and TK1 or CCNA2, GGH, CCNB1, MELK and HMMR or EIF2S1, TEAD4, HMGA1, RIMS2, and H2AFZ).

In another embodiment, the biomarkers of the invention include any one, or any combination, of the following genes: CBX7, TMPRSS2, GPR116, KCNJ15, PTPN13, CTSH, PPFIBP2, CD302, SFTPB, HSD17B6, DLC1, ADRB2, PARM1, KLRB1, MS4A1, STX1A, KLK6, SLC16A3, PYGL, LDHA, ITGA5, VEGFC, EEF1A2, TPX2, UCK2, PHKA1, EIF4A3, TK1, CCNA2, GGH, CCNB1, MELK, HMMR, EIF2S1, TEAD4, HMGA1, RIMS2, H2AFZ. In another embodiment, the biomarkers of the invention include at least 15, 20, 25, 30, 35, 36, 37 or 38 of the following genes: CBX7, TMPRSS2, GPR116, KCNJ15, PTPN13, CTSH, PPFIBP2, CD302, SFTPB, HSD17B6, DLC1, ADRB22, PARM1, KLRB1, MS4A1, STX1A, KLK6, SLC16A3, PYGL, LDHA, ITGA5, VEGFC, EEF1A2, TPX2, UCK2, PHKA1, EIF4A3, TK1, CCNA2, GGH, CCNB1, MELK, HMMR, EIF2S1, TEAD4, HMGA1, RIMS2, H2AFZ. In a particular embodiment, the following 37 biomarkers are selected: CBX7, TMPRSS2, GPR116, KCNJ15, PTPN13, CTSH, PPFIBP2, CD302, SFTPB, HSD17B6, DLC1, ADRB2, PARM1, KLRB1, MS4A1, STX1A, KLK6, SLC16A3, PYGL, LDHA, ITGA5, VEGFC, TPX2, UCK2, PHKA1, EIF4A3, TK1, CCNA2, GGH, CCNB1, MELK, HMMR, EIF2S1, TEAD4, HMGA1, RIMS2, H2AFZ.

In one example, the expression profile can be a set of values representing mRNA levels of one or more biomarkers listed in Table 1, Table 2, and/or Table 3. In another example, the expression profile can include a set of values representing one or more protein or polypeptides encoded by the biomarkers listed in Table 1, Table 2, and/or Table 3.

### Preparation of Samples

Any appropriate test sample of cells taken from an individual having early stage lung cancer who has undergone a surgical resection can be used to determine the expression of a plurality of biomarkers of the invention. The type and classification of the early stage lung cancer can vary. The lung cancer can be in Stage I and/or Stage II. The test sample can be a non-small cell lung cancer (NSCLC) which includes squamous cell carcinoma, adenocarcinoma, large cell carcinoma, as well as all histotypes irrespective of the subgroup.

Generally, the test sample of cells or tissue sample will be obtained from the subject with cancer by biopsy or surgical resection. The surgical resection can be curative or non-curative/RO. A sample of cells, tissue, or fluid may be removed by needle aspiration biopsy. For this, a fine needle attached to a syringe is inserted through the skin and into the organ or tissue of interest. The needle is typically guided to the region of interest using ultrasound or computed tomography (CT) imaging. Once the needle is inserted into the tissue, a vacuum is created with the syringe such that cells or fluid may be sucked through the needle and collected in the syringe. A sample of cells or tissue may also be removed by incisional or core biopsy. For this, a cone, a cylinder, or a tiny bit of tissue is removed from the region of interest. CT imaging, ultrasound, or an endoscope is generally used to guide this type of biopsy. More particularly, the entire cancerous lesion may be removed by excisional biopsy or surgical resection. In the present invention, the test sample is typically a sample of cells removed as part of surgical resection.

The test sample of, for example tissue, may also be stored in, e.g., RNAlater (Ambion; Austin Tex.) or flash frozen and stored at -80°C. for later use. The biopsied tissue sample may also be fixed with a fixative, such as formaldehyde, paraformaldehyde, or acetic acid/ethanol. The fixed tissue sample may be embedded in wax (paraffin) or a plastic resin. The embedded tissue sample (or frozen tissue sample) may be cut into thin sections. RNA or protein may also be extracted from a fixed or wax-embedded tissue sample.

The subject with cancer will generally be a mammalian subject such as a primate. In an exemplary embodiment, the subject is a human.

Once a sample of cells or sample of tissue is removed from the subject with cancer, it may be processed for the isolation of RNA or protein using techniques well known in the art and as described below.

In one example, RNA may be extracted from tissue or cell samples by a variety of methods, for example, guanidium thiocyanate lysis followed by CsCl centrifugation (Chirgwin, et al., Biochemistry 18:5294-5299, 1979). RNA from single cells may be obtained as described in methods for preparing cDNA libraries from single cells (see, e.g., Dulac, Curr. Top. Dev. Biol. 36:245, 1998; Jena, et al., J. Immunol. Methods 190:199, 1996). The RNA sample can be further enriched for a particular species. In one embodiment, for example, poly(A)+ RNA may be isolated from an RNA sample. In particular, poly -T oligonucleotides may be immobilized on a solid support to serve as affinity ligands for mRNA. Kits for this purpose are commercially available, for example, the MessageMaker kit (Life Technologies, Grand Island, N. Y.). In one embodiment, the RNA population may be enriched for sequences of interest, as detailed on Tables 1-3. Enrichment may be accomplished, for example, by primer-specific cDNA synthesis, or multiple rounds of linear amplification based on cDNA synthesis and template-directed in vitro transcription (see, e.g., Wang, et al., Proc. Natl. Acad. Sci. USA 86:9717, 1989).

### Detection of expression of the biomarker

In one example, the method includes determining expression of one or more biomarkers listed in Table 1, Table 2, and/or Table 3, or their gene products from a tumor sample of a test cancer patient. The gene sequences of each of the biomarkers listed in Table 1, Table 2, and/or Table 3 can be detected using methods known in the art, e.g., agents that can be used to specifically detect the gene or gene products thereof.

Exemplary detection agents are nucleic acid probes, which hybridize to nucleic acids corresponding to the genes disclosed herein, and antibodies which bind to the encoded products of these genes. The biomarkers listed in Table 1, Table 2, and/or Table 3 are intended to also include naturally occurring sequences including allelic variants and other family members. The biomarkers of the invention also include sequences that are complementary to those listed sequences resulting from the degeneracy of the code and also sequences that are sufficiently homologous and sequences which hybridize under stringent conditions to the biomarkers listed in Table 1, Table 2, and/or Table 3.

In one embodiment, the method includes: providing a nucleic acid probe comprising a nucleotide sequence, for example, at least 10, 15, 25 or 40 nucleotides, and up to all or nearly all of the coding sequence which is complementary to a portion of the coding sequence of a nucleic acid sequence listed in Table 1, Table 2, and/or Table 3; obtaining a tissue sample from a mammal having a cancerous cell; contacting the nucleic acid probe under stringent conditions with RNA obtained from a biopsy taken from a patient with NSCLC (e.g., in a Northern blot or in situ hybridization assay); and determining the amount of hybridization of the probe with RNA.

Conditions for hybridization are known to those skilled in the art and can be found in Current Protocols in Molecular Biology, John Wiley and Sons, N.Y. (1989), 6.3.1-6.3.6. A preferred, non-limiting example of highly stringent hybridization conditions are hybridization in 6 X sodium chloride/sodium citrate (SSC) at about 45 degrees centigrade followed by one or more washes in 0.2 X SSC, 0.1 percent SDS at 50-65 degrees centigrade. By "sufficiently homologous" it is meant a amino acid or nucleotide sequence of a biomarker which contains a sufficient or minimum number of identical or equivalent (e.g., an amino acid residue which has a similar side chain) amino acid residues or nucleotides to a second amino acid or nucleotide sequence such that the first and second amino acid or nucleotide sequences share common structural domains or motifs and/or a common functional activity. For example, amino acid or nucleotide sequences which share common structural domains have at least about 50 percent homology, at least about 60 percent homology, at least about 70 percent, at least about 80 percent, and at least about 90-95 percent homology across the amino acid sequences of the domains are defined herein as sufficiently homologous. Furthermore, amino acid or nucleotide sequences at least about 50 percent homology, at least about 60-70 percent homology, at least about 70-80 percent, at least about 80-90 percent, and at least about 90-95 percent and share a common functional activity are defined herein as sufficiently homologous.

The comparison of sequences and determination of percent homology between two sequences can be accomplished using a mathematical algorithim. A preferred, non-limiting example of a mathematical algorithim utilized for the comparison of sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs (version 2.0) of Altschul, et al. (1990) J. MoI. Biol. 215:403-10. BLAST nucleotide searches can be performed with the NBLAST program, score=100, wordlength=12 to obtain nucleotide sequences homologous to TRL nucleic acid molecules of the invention. BLAST protein searches can be performed with the XBLAST program, score=50, wordlength=3 to obtain amino acid sequences homologous to the protein sequences encoded by the biomarkers listed in Table 1, Table 2, and/or Table 3. To obtain gapped alignments for comparison purposes, Gapped BLAST can be utilized as described in Altschul et al., (1997) Nucleic Acids Research 25(17):3389-3402. When utilizing BLAST and Gapped BLAST programs, the default parameters of the respective programs (e.g., XBLAST and NBLAST) can be used. See http://www.ncbi.nlm.nih.gov. Another preferred, non-limiting example of a mathematical algorithim utilized for the comparison of sequences is the ALIGN algorithm of Myers and Miller, CABIOS (1989). When utilizing the ALIGN program for comparing amino acid sequences, a PAM120 weight residue table, a gap length penalty of 12, and a gap penalty of 4 can be used.

Nucleic acids may be labeled during or after enrichment and/or amplification of RNAs. For example, reverse transcription may be carried out in the presence of a dNTP conjugated to a detectable label, for example, a fluorescently labeled dNTP. In another embodiment, the cDNA or RNA probe may be synthesized in the absence of detectable label and may be labeled subsequently, for example, by incorporating biotinylated dNTPs or rNTP, or some similar means (e.g., photo-cross-linking a psoralen derivative of biotin to RNAs), followed by addition of labeled streptavidin (e.g., phycoerythrin-conjugated streptavidin) or the equivalent.

Fluorescent moieties or labels of interest include coumarin and its derivatives (e.g., 7- amino-4-methylcoumarin, aminocoumarin); bodipy dyes such as Bodipy FL and cascade blue; fluorescein and its derivatives (e.g., fluorescein isothiocyanate, Oregon green); rhodamine dyes (e.g., Texas red, tetramethylrhodamine); eosins and erythrosins; cyanine dyes (e.g., Cy2, Cy3, Cy3.5, Cy5, Cy5.5, Cy7); FluorX, macrocyclic chelates of lanthanide ions (e.g., quantum dye.TM.); fluorescent energy transfer dyes such as thiazole orange-ethidium heterodimer, TOTAB, dansyl, etc. Individual fluorescent compounds which have functionalities for linking to an element desirably detected in an apparatus or assay of the invention, or which may be modified to incorporate such functionalities may also be utilized (see, e.g., Kricka, 1992, Nonisotopic DNA Probe Techniques, Academic Press San Diego; Calif.). Chemiluminescent labels include luciferin and 2,3-dihydrophthalazinediones, for example, luminol.

### Detecting expression of the biomarker gene product

Detecting for the presence of a protein product encoded by one or more of the biomarkers listed in Table 1, Table 2 and/or Table 3 can be done by using any appropriate method known in the art. For example, an agent of interest that can be used to detect a particular protein of interest, for example using an antibody. The method for producing polyclonal and/or monoclonal antibodies that specifically bind to polypeptides useful in the present invention is known to those of skill in the art and may be found in, for example, Dymecki, et al., (J. Biol. Chem. 267:4815, 1992); Boersma and Van Leeuwen, (J. Neurosci. Methods 51:317, 1994); Green, et al., (Cell 28:477, 1982); and Arnheiter, et al., (Nature 294:278, 1981). In one embodiment, an immunoassay can be used to quantitate the levels of proteins in cell samples. The invention is not limited to a particular assay procedure, and therefore, is intended to include both homogeneous and heterogeneous procedures. Exemplary immunoassays that may be conducted according to the invention include fluorescence polarization immunoassay (FPIA)₅ fluorescence immunoassay (FIA), enzyme immunoassay (EIA), nephelometric inhibition immunoassay (NIA), enzyme-linked immunosorbent assay (ELISA), and radioimmunoassay (RIA). An indicator moiety, or label group, may be attached to the subject antibodies and is selected so as to meet the needs of various uses of the method that are often dictated by the availability of assay equipment and compatible immunoassay procedures. General techniques to be used in performing the various immunoassays noted above are known to those of ordinary skill in the art. Alternatively other methods can be used such as Western blot analysis that includes electrophoretically separating proteins on a polyacrylamide gel, and after staining the separated proteins, the relative amount of each protein can be quantified by assessing its optical density. Alternatively, other methods such as dot-blot assays, FACS or immunohistochemistry can be used.

The tissue samples are fixed by treatment with a reagent such as formalin, glutaraldehyde, methanol, or the like. The samples are then incubated with an antibody (e.g., a monoclonal antibody) with binding specificity for the marker polypeptides. This antibody may be conjugated to a label for subsequent detection of binding. Samples are incubated for a time sufficient for formation of the immunocomplexes. Binding of the antibody is then detected by virtue of a label conjugated to this antibody. Where the antibody is unlabeled, a second labeled antibody may be employed, for example, that is specific for the isotype of the anti-marker polypeptide antibody. Examples of labels that may be employed include radionuclides, fluorescers, chemiluminescers, enzymes, and the like.

Where enzymes are employed, the substrate for the enzyme may be added to the samples to provide a colored or fluorescent product. Examples of suitable enzymes for use in conjugates include horseradish peroxidase, alkaline phosphatase, malate dehydrogenase, and the like. Where not commercially available, such antibody-enzyme conjugates are readily produced by techniques known to those skilled in the art.

In yet another embodiment, the invention contemplates using a panel of antibodies that are generated against the marker polypeptides of this invention.

### mRNA detection

An important aspect of the present invention is to measure the expression level of one or more biomarkers identified in Table 1, Table 2 and/or Table 3 in a lung cancer tumor biopsy taken from a subject suffering from early stage lung cancer following surgical resection. The expression levels can be analyzed and used to generate a risk score.

In one example, reverse Transcriptase PCR (RT-PCR) can be used for gene expression profiling to compare mRNA levels in different sample populations. The method includes isolating mRNA using any technique known in the art, e.g., by using a purification kit, buffer set and protease from commercial manufacturers, such as Qiagen, according to the manufacturer's instructions or complete DNA and RNA Purification Kit (EPICENTRE(R), Madison, WT). The reverse transcription step is typically primed using specific primers, random hexamers, or oligo-dT primers, depending on the circumstances and the goal of expression profiling and the cDNA derived can then be used as a template in the subsequent PCR reaction. TaqMan(R) RT-PCR can then be performed using, e.g., commercially available equipment.

A more recent variation of the RT-PCR technique is the real time quantitative PCR, which measures PCR product accumulation through a dual-labeled fluorigenic probe (i.e., TaqMan(R) probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al, Genome Research 6:986-994 (1996).

In another example, microarrays are used which include one or more probes corresponding to one or more of biomarkers identified in Table 1, Table 2 and/or Table 3. The method described above results in the production of hybridization patterns of labeled target nucleic acids on the array surface. The resultant hybridization patterns of labeled nucleic acids may be visualized or detected in a variety of ways, with the particular manner of detection selected based on the particular label of the target nucleic acid. Representative detection means include scintillation counting, autoradiography, fluorescence measurement, calorimetric measurement, light emission measurement, light scattering, and the like.

In one example, the method of detection utilizes an array scanner that is commercially available (Affymetrix, Santa Clara, Calif.), for example, the 417Arrayer, the 418Array Scanner, or the Agilent GeneArray.Scanner. This scanner is controlled from a system computer with an interface and easy-to-use software tools. The output may be directly imported into or directly read by a variety of software applications.

As used herein, the control for comparison can be determined by one skilled in the art. In one aspect, the control is determined by choosing a value that serves as a cut-off value. For example, the value can be a value that differentiates between e.g., those test samples that have good survival and those that have bad survival; or between those test samples where the individual would benefit from adjuvant therapy and those that would not; or between those test samples where the individual would benefit form the administration of a particular drug such as an inhibitor of angiogenesis or an inhibitor of proliferation. A patient that might benefit from adjuvant therapy means an improvement in any measure of patient status including those measures ordinarily used in the art such as overall survival, long-term survival, recurrence-free survival, and distant recurrence-free survival.

Other methods for determining levels of gene expression include MassARRAY-based gene expression profiling method, developed by Sequenom, Inc. (San Diego, CA) and serial analysis of gene expression (SAGE) (Velculescu et al, Science 270:484-487 (1995); and Velculescu et al, Cell 88:243-51 (1997).

Yet other methods for determining levels of gene expression in FFPE materials are qNPA™ technology (HTG Molecular Diagnostics, Inc., Arizona) and nanoString™ Technologies (Seattle), where neither RNA extraction nor amplification are required. Using qNPA technology the FFPE sample is first exposed to the HTG lysis buffer and nuclease protection probes complementary to the mRNA of the biomarkers described herein are then added to the solution. The probes hybridize to all RNA biomarkers of interest, soluble and cross-linked. After hybridization, S1 nuclease is added destroying all nonspecific, single stranded nucleic acids, producing a stoichiometric amount of biomarker-mRNA probe duplexes. Base hydrolysis then releases the probe from the duplexes. Probes can then be transferred to a programmed ArrayPlate, detection linker added, and both probes and detection linkers captured onto the array. The ArrayPlate is then washe and a HRP-labeled detection probe added, incubated. The array plate is then washed and a chemiluminescent substrate added. Finally, the ArrayPlate is imaged and expression of each of the biomarkers in all wells measured. Using Nanostring technologies two ∼50 base probes per biomarker mRNA are employed which hybridize to the mRNA in solution. The reporter probe carries the signal, while the capture probe allows the complex to be immobilized for data collection. Following hybridization, excess probes are removed and the probe/target complexes are aligned and immobilized in a Counter Cartridge.

In the method of the invention the level of expression of one or more biomarkers as described above is measured and analyzed and used to generate a risk score as described below. The expression threshold can be used prognostically, e.g., to select for those individuals who have good survival and those that have bad survival.

It is necessary to correct for (normalize away) both differences in the amount of RNA assayed and variability in the quality of the RNA used. Therefore, the assay typically measures and incorporates the expression of certain normalizing genes, including well known housekeeping genes, such as GAPDH and Cypl. Alternatively, normalization can be based on the mean or median signal (Ct) of all of the assayed biomarkers or a large subset thereof (global normalization approach). On a gene-by-gene basis, measured normalized amount of a patient tumor mRNA is compared to the amount found in a lung cancer tissue reference set. The number (N) of lung cancer tissues in this reference set should be sufficiently high to ensure that different reference sets (as a whole) behave essentially the same way. If this condition is met, the identity of the individual lung cancer tissue present in a particular set will have no significant impact on the relative amounts of the biomarkers assayed.

In the methods of the invention, the expression of each biomarker is measured and typically will be converted into an expression value. These expression values then will be used to generate a risk score by weighted averaging. The risk score is associated to risk of death, metastasis or relapse through a calibration database, either through parametric formula or non-parametric, data-driven models. This database is constructed from a reference set of sample with known expression values, risk scores and clinical follow up. The risk score calibration may be available separately for each module (table 1, 2 or 3) or specific to a particular disease subtype as defined by histology, tumor staging or other characteristics such as patient age. For treatment response prediction, separate calibration formulae or databases are constructed for patients treated by specific therapies (or no treatment). A compound risk score (combining modules in table 1, 2, or 3 with certain weighting) may also be used, with its own calibration formula or database. Clinical decision making protocol may be done according to the calibrated risk score or predicted survival or time to events (relapse or metastasis) as described above.

The risk score once calculated may also be used to decide upon an appropriate course of treatment for the subject. A subject having a high risk score (i.e., short survival time or poor prognosis) may benefit from receiving adjuvant therapy. Adjuvant therapy may include appropriate chemotherapy agents, e.g., Paraplatin (carboplatin), Platinol (cisplatin), Taxotere (docetaxel), Adriamycin (doxorubicin), VePesid (etoposide), Gemzar (gemcitabine), Ifex (ifosfamide), Camptosar (irinotecan), Taxol (paclitaxel), Alimta (pemetrexed) and Hycamtin (topotecan) and/or radiation therapy. A subject having a negative risk score (i.e., long survival time or good prognosis) may not benefit from additional treatment.

In another example, the risk score generated using a gene set from a particular Table can be used to determine a course of specific therapy. For example, if an individual has a high risk score based on analysis of the biomarkers of Table 2 that individual may benefit from receiving adjuvant therapy which includes an angiogenesis inhibitor such as avastin, srafinib, sunitinib or pazopanib. Alternatively, if an individual has a high risk score based on analysis of biomarkers of Table 3 that individual may benefit from receiving adjuvant therapy which includes an anti-proliferative agent such as a topoisomerase inhibitor (I & II), Taxane, anthracycline, antitublin, antimetabolite or alkylating agents.

### Data analysis

To facilitate the sample analysis operation, the data obtained by the reader from the device may be analyzed using a digital computer. Typically, the computer will be appropriately programmed for receipt and storage of the data from the device, as well as for analysis and reporting of the data gathered, for example, subtraction of the background, verifying that controls have performed properly, normalizing the signals, interpreting fluorescence data to determine the amount of hybridized target, normalization of background, and the like.

### Kits

The invention further provides kits for determining the expression level of the biomarkers described herein. The kits may be useful for determining prognosis of lung cancer subjects. A kit can comprise a microarray comprising probes of any, of any combinations of biomarkers, identified in Tables 1-3 and/or any other solid support to which probes can be attached and the solid support can be used to measure gene expression of a test sample. In one embodiment, the kit comprises a computer readable medium which includes expression profile analysis software capable of being loaded into the memory of a computer system and which can convert the measured expression values into a risk score. A kit may further comprise nucleic acid controls, buffers, and instructions for use.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

### Examples

### Example 1:

A combination of a Novartis dataset and various public data sets from cohorts of lung patients was studied. Description of the patient selection criteria and clinical characteristics can be found in the respective original articles for the public data sets (see below). For the Novartis dataset, 412 patient samples where collected from NSCLC patients who had undergone surgical resection. Standard staging procedures were performed including CT-Scans, FDG-PET of suspicious Lymphnodes (> 1 cm in CT) and MRI. NSCLC histologies was performed to determine if NSCLC was Squamous, Adeno-Carcinoma or others such as BAC. TNM-based staging was also performed to define whether NSCLC was Stage I or Stage II. The fresh frozen tissue was banked for genomic analysis. The primary endpoint was overall survival. Overall survival refers to the time (in years) from first surgery and can be defined by a period such as at least 3 years, for example a 5 year period, which is relapse or recurrence free.

Public datasets used in this example:

**Table 5**

| **Dataset Name** | **Institution** | **Reference** | **Source** | **Expression Microarray Platform** |
|---|---|---|---|---|
| DFCI | Dana-Farber Cancer Institute | Shedden et al 2008 | caArray | Affymetrix U133A |
| | | | https://array.nci.nih.gov/caarray/project/ jacob-00182 | |
| HLM | Moffitt Cancer Center | Shedden et al 2008 | caArray | Affymetrix U133A |
| | | | https://array.nci.nih.gov/caarray/project/ jacob-00182 | |
| JBR | Ontario Cancer Institute, Prince Margaret Hospital | Zhu et al 2010 | GEO GSE14814 | Affymetrix U133A |
| | | | http://www.ncbi.nlm.nih.gov/geo/query/ acc.cgi?acc=GSE14814 | |
| MI | University of Michigan Cancer Center | Shedden et al 2008 | caArray | Affymetrix U133A |
| | | | https://array.nci.nih.gov/caarray/project/ jacob-00182 | |
| MIT | Massachusetts Institute of Technology | Bhattacharjee et al 2001 | http://broad.institute.org/mpr/lung | Affymetrix U95A |
| MSKCC | Memorial Sloan-Kettering Cancer Center | Shedden et at 2008 | caArray | Affymetrix U133A |
| | | | https://array.nci.nih.gov/caarray/project/ jacob-00182 | |
| NCCH | University of Caroline at Chapel Hill | Wilkerson et al 2010 | GEO GSE17710 | Agilent UNC custom array 44k |
| | | | http://www.ncbi.nlm.nih.gov/geo/query/ acc.cgi?acc=GSE17710 | |
| NU1 | Nagoya University | Takeuchi et al 2006 | GEO GSE11969 | Agilent custom array 21.6k |
| | | | http://www.ncbi.nlm.nih.gov/geo/query/ acc.cgi?acc=GSE11969 | |
| VRX | Veridex, LLC | Raponi et al 2006 | GEO GSE4573 | Affymetrix U133A |
| | | | http://www.ncbi.nlm.nih.gov/geo/query/ acc.cgi?acc=GSE4573 | |
| WU | Washington University | Lu et al 2006 | GEO GSE6253 | Affymetrix U133A, U133B, U95Av2 |
| | | | http://www.ncbi.nlm.nih.gov/geo/query/ acc.cgi?acc=GSE6253 | |

*Dataset references* include:
Bhattacharjee A et al. *Proc Natl Acad Sci USA* **98**:13790-5, 2001; Takeuchi et al..(2006) *J Clin Oncol* **24**:1679-88; Raponi et al. (2006) *Cancer Res* **66**:7466-72; Lu et al. (2006) *PLoS Med* **3**:e467; Shedden et al. (2008) *Nat Med* **14**:822-7; Hou et al. (2010) *PLoS One* **5**:e10312; Wilkerson et al. (2010) *Clin Cancer Res* **16**:4864-75; Zhu et al. (2010) *J Clin Oncol* **28**:4417-24

### Processing of the gene expression microarray of the public datasets

The procedure for preparing the datasets for the analysis was similar to.that described in Wirapati et. al. 2008 *Breast Cancer Research* **10**:R65, and briefly outlined as follow:
1. For gene expression data, the normalized or preprocessed expression values that was provided by the original authors was used without modification
2. Probes or probesets in the microarray platform were remapped to the same reference sequence database (RefSeq version 39)
3. Only patients with early stage (stage I and II) were used in this example, totalling n=834 from all datasets (with n=571 of stage I, and n=263 of stage II). All histologic types are included, although most are adenocarcinoma (n=585) or squamous cell carcinoma (n=226), with only n=23 from other histologic types.

The signature genes were identified by large-scale integrated analysis of a comprehensive gene expression and clinical database consisting of lung cancer datasets newly generated by Novartis (two cohorts totalling 412 patients, unpublished) and publicly available gene expression datasets. Signature genes were selected and grouped them into the three modules (Table 1,2, and 3) based on criteria including similarity of expression patterns with those of other types of cancer and biological function. Publicly available datasets were chosen such that the prognostic performance could be independently verified using the methods outlined below.

When applying a biomarker signature (a set of biomarkers as specified by Tables 1,2, and/or 3), the genes that were missing were ignored. A raw score was assigned to each signature by averaging the expression values for genes that were present in a particular platform. The standardized score was produced by subtracting the mean of the raw score, and dividing by the standard deviation. The mean and standard deviation was determined separately for each dataset. Three different scores were produced for each patient. We will refer to them as modules 1,2, or 3, corresponding to the gene sets in Table 1, 2, or 3, respectively.

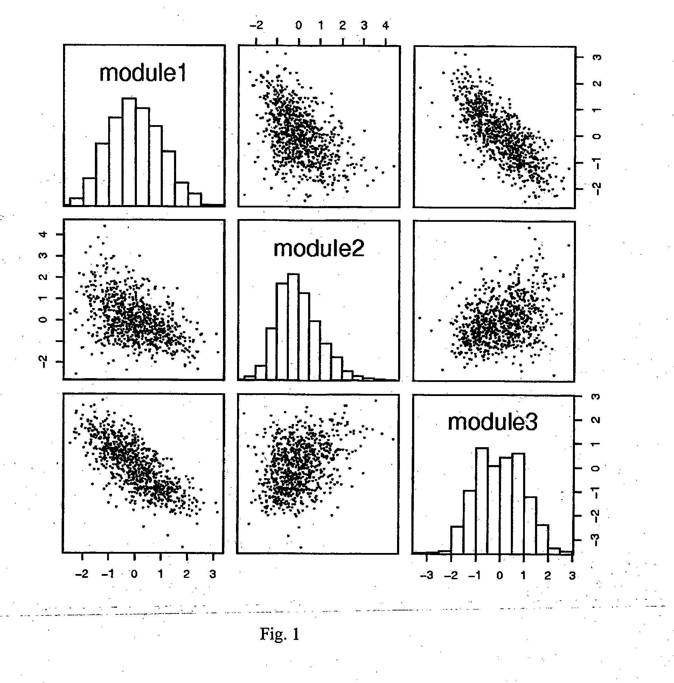

To demonstrate that the scores from the three modules were not providing similar information, we did scatter plots of pairwise distributions of the scores in Fig. 1. Module 1 is acting in roughly the opposite direction of module 3, in accordance with their biological involvement in tumor suppression (module 1) and proliferation (module 3). Although these two modules may provide very similar prognostic information, the reciprocity of the expression was important technically for accurate detection. Thus a low value from one module was only interpreted as low average expression (rather than technical failure in detection) if it was accompanied by high value of the other module. Module 2 showed slight correlation with module 1 and 3, but with interesting asymmetric patterns: although it was possible to have both low values of module 1 and 2, it was unlikely to have high values in both. On the other hand, high values of module 2 seems to require high value of module 3, although the converse is not true (i.e., some tumors with high value of module 3 may have low value of module 2).

### Assessment of the prognostic performance of the signatures

To show the clinical utility of each of the module scores, we performed survival analysis using Kaplan-Meier curves (Kaplan and Meier J. Am. Statist. Assoc. 53:457-481, 1953), using the quantitative scores to divide the patients into quartiles (groups containing 25% of the patients).

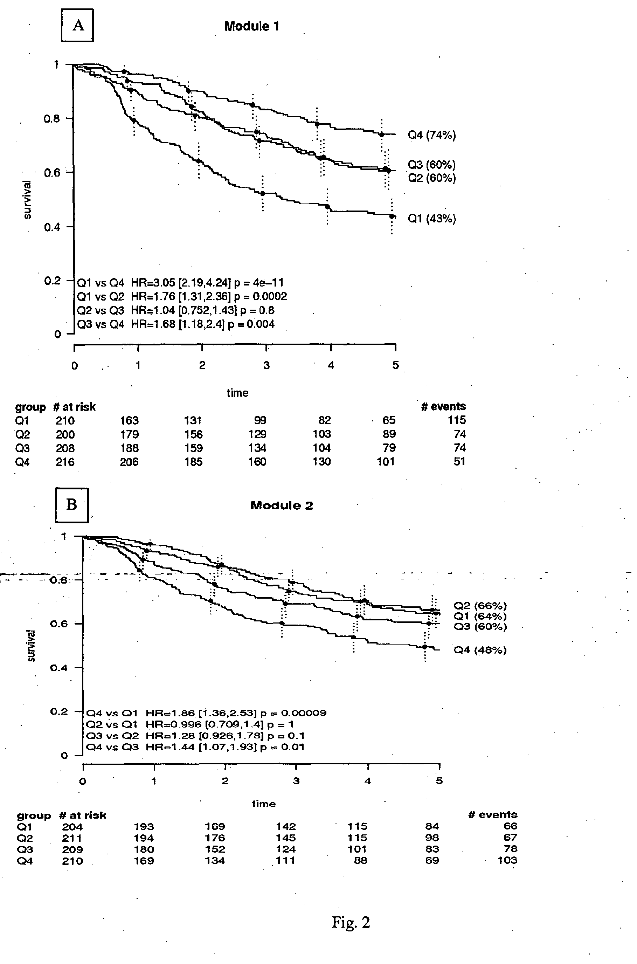

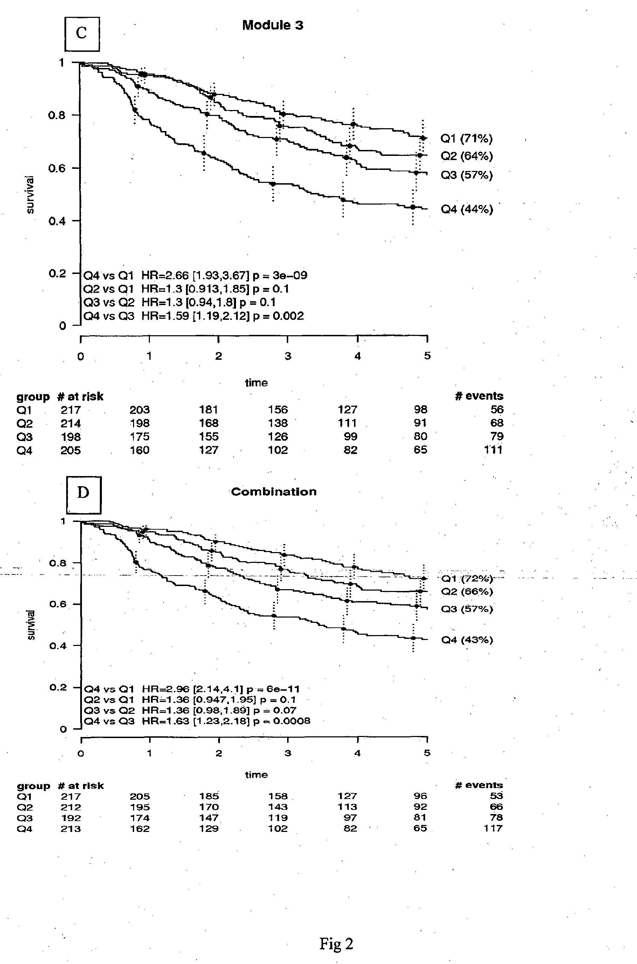

Fig 2A-C show the prognostic performance of the three signatures (module 1, 2, and 3) when they are applied separately, shown by Kaplan-Meier survival analysis of the overall survival of the patients up to five years after surgery. Patients are categorized into four groups Q1, Q2, Q3, and Q4, according to the quartiles of the scores, corresponding to the 1st, 2nd, 3rd, and 4th quartiles, respectively. This categorization allows examination of the change of risk as function of the score. Cox regression analysis is used to compare selected pairs of curves, with the results showing the hazard ratio (HR) and its 95% confidence interval, and the p-value of hypothesis test against no effect (HR = 1). The numbers to the right of each curve are five year survival in percent. The first three panels demonstrate that each signature has a prognostic value on its own. Fig. 2D ("Combination") shows the results of a risk score obtained by simple combination of the three modules ( -module1 + module2 + module3 )

An example of a prognostic system combining the three score is also shown in Fig. 2D. The scores were simply summed, with multiplication by -1 for module 1 to invert the direction of the effect. Although no dramatic improvement over some individual modules was seen, the gradation of the risk as a function of the score was more even.

In summary, each individual signature (module 1, 2 or 3), as well as their combination (module 1,2 and 3), showed the ability to distinguish the survival (or equivalently, disease-related mortality) of subgroups of patients. In all cases substantial and statistically significant differences are observed at least between the extreme quartiles.

### Performance of the signatures in the context of clinico-pathologic factors

To show that the proposed signatures add new prognostic information to well-established factors such as histology, tumor staging and age at diagnosis, we performed similar analyses as above, but stratifying the data into groups. In this illustrative example, we only showed the stratification by each factor separately, dividing them into two major groups in order to have sufficient sample size in each group. The factors considered are:
- Tumor staging: stage I versus stage II
- Histology: adenocarcinoma versus squamous cell carcinoma
- Age at diagnosis: less-than-or-equal to 65 year versus greater than 65 year (this is the median age of the patients in the data)

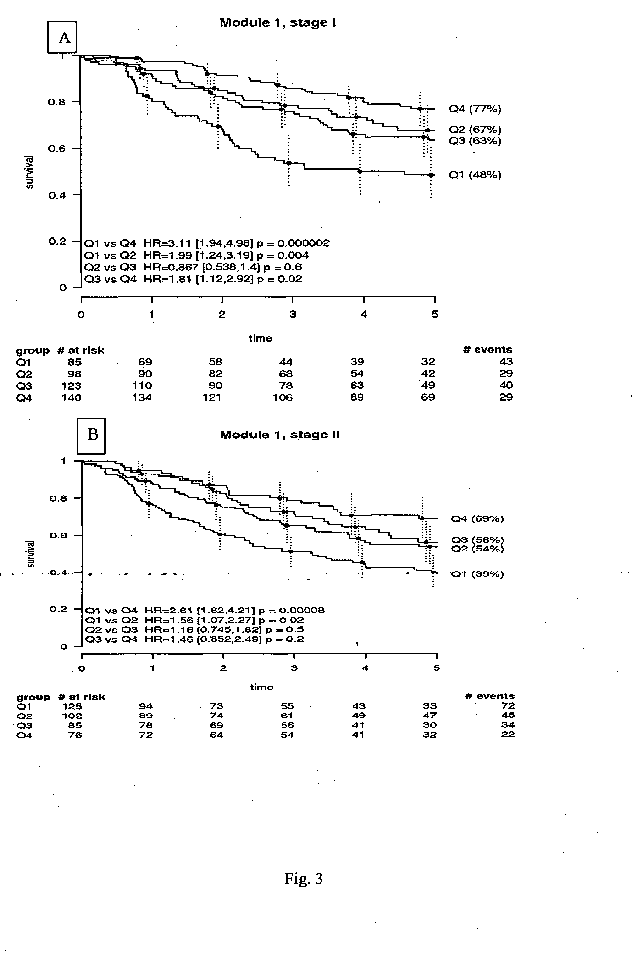

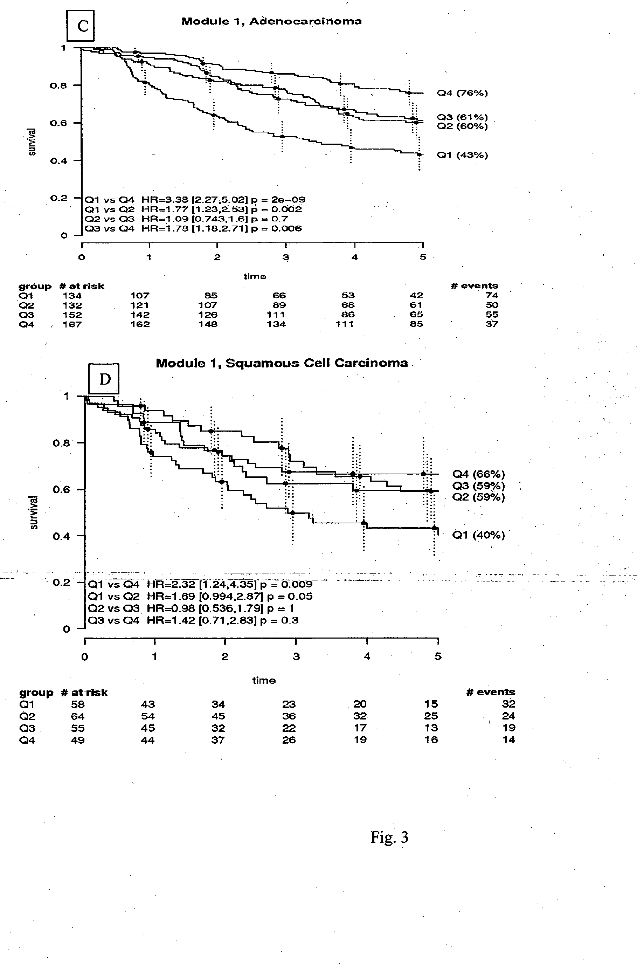

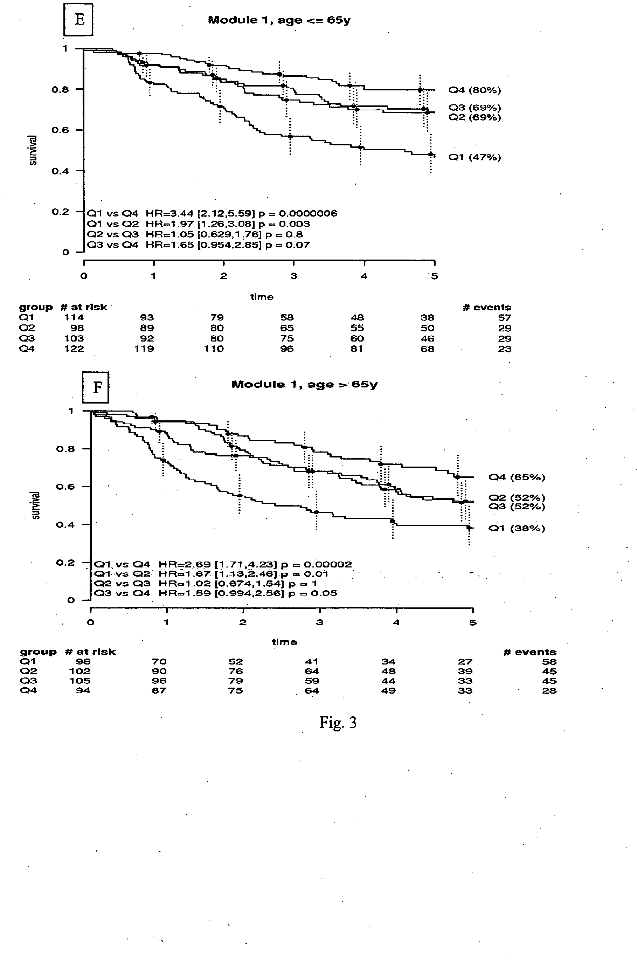

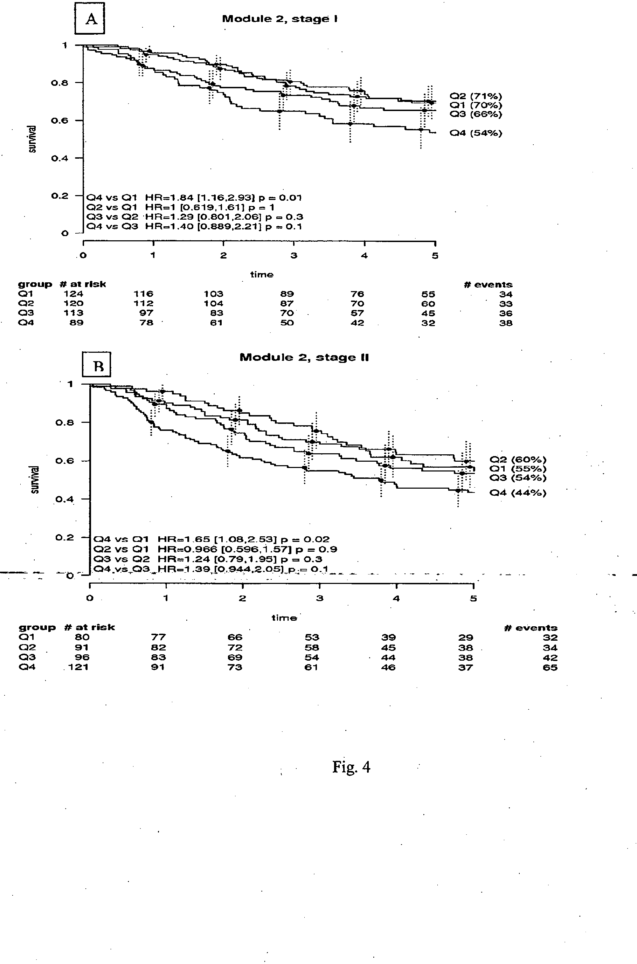

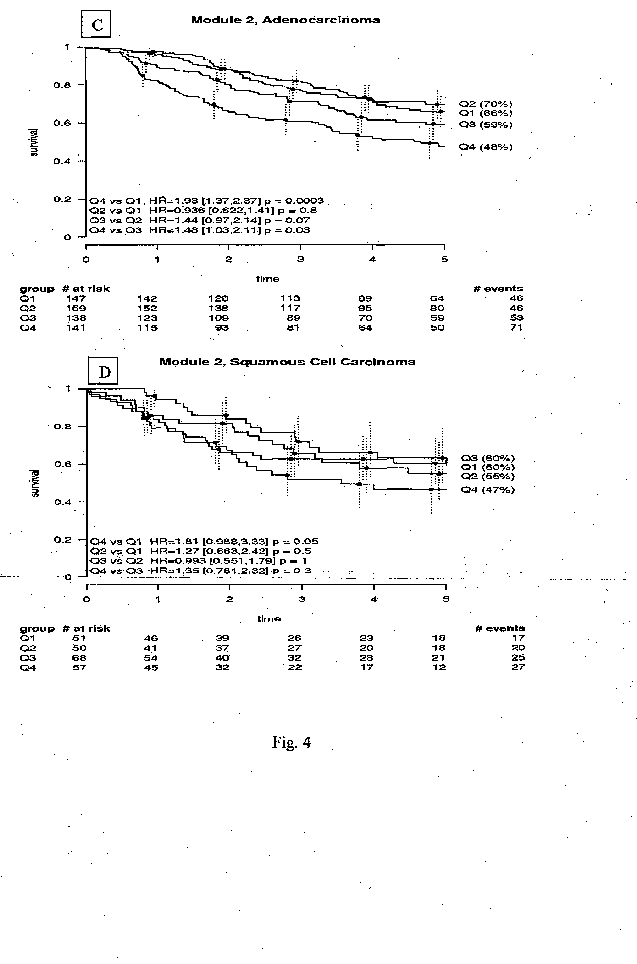

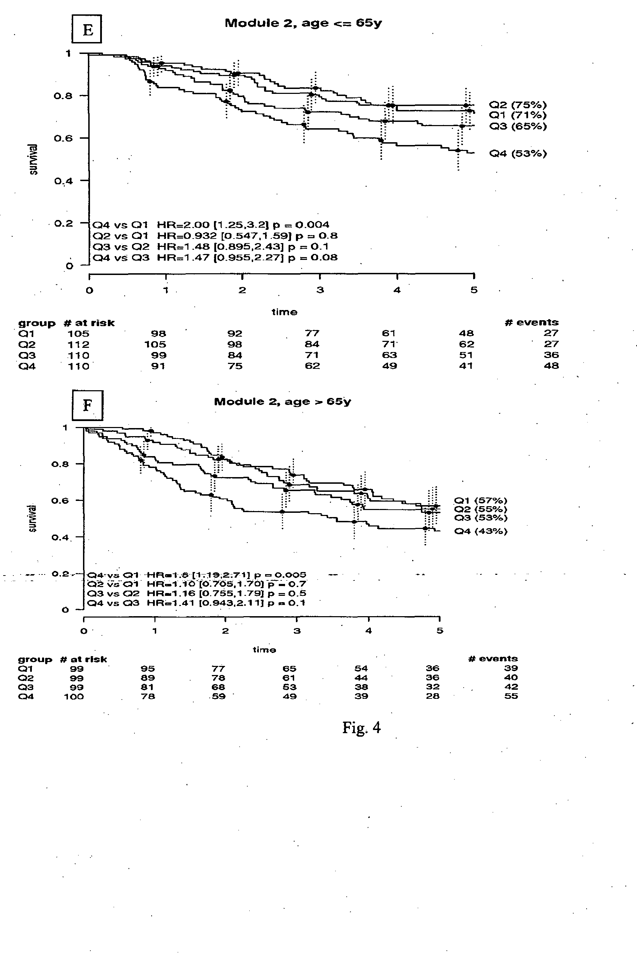

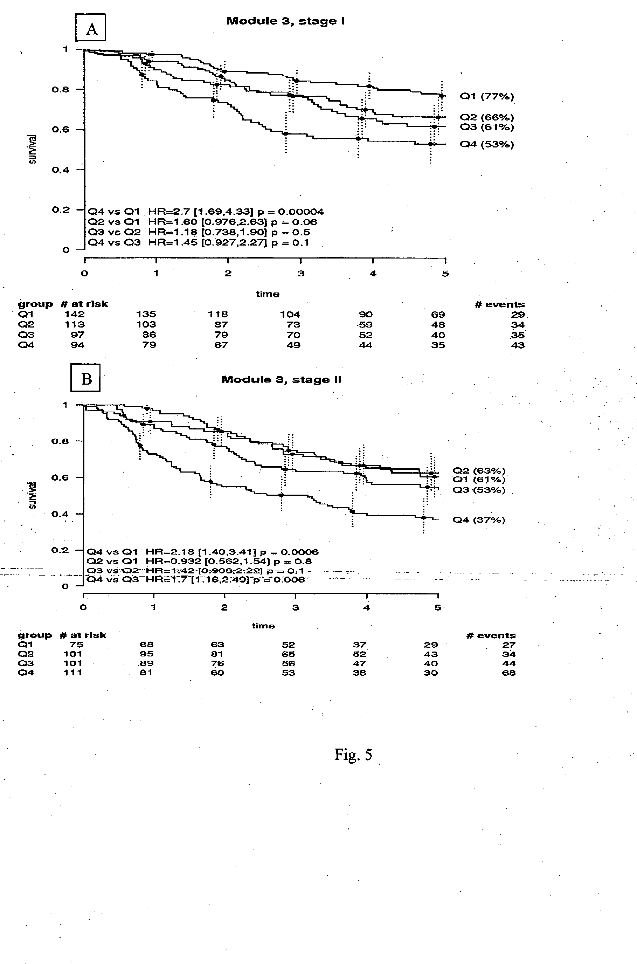

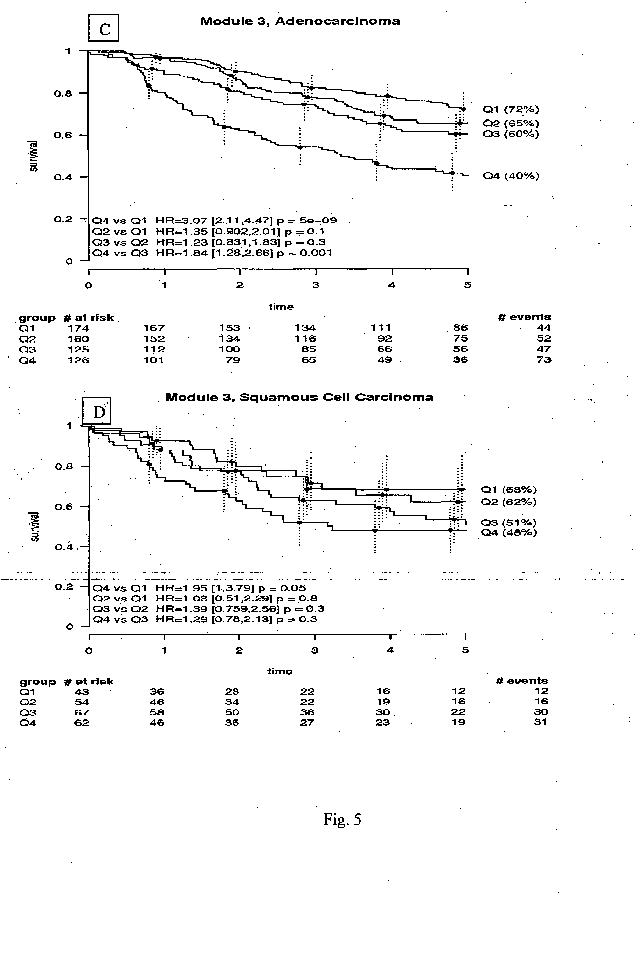

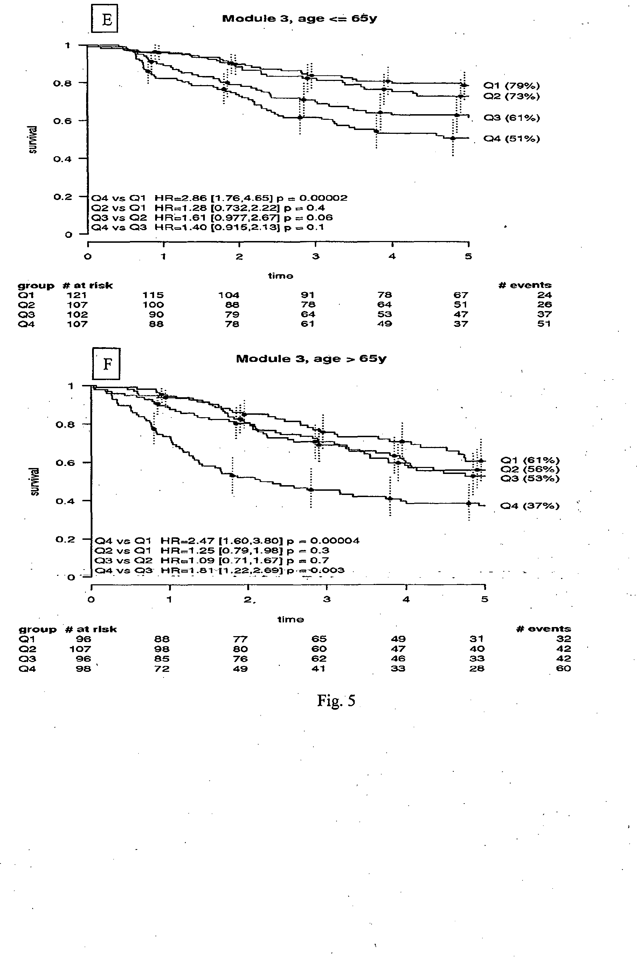

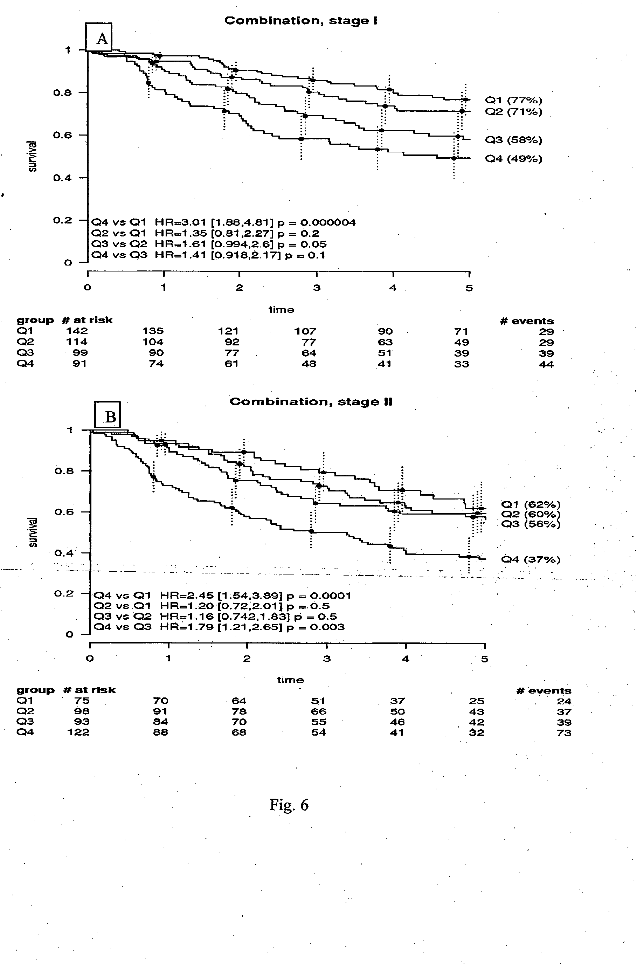

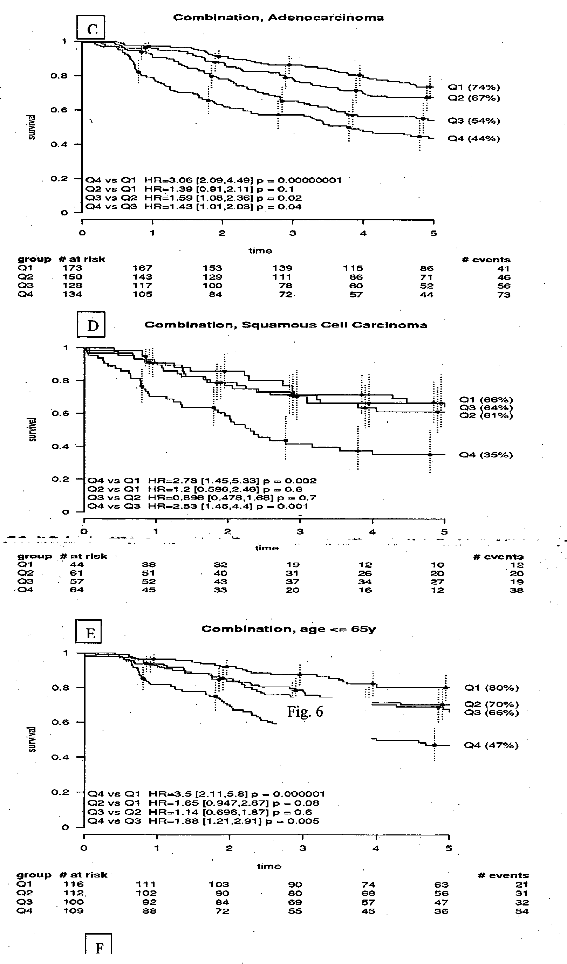

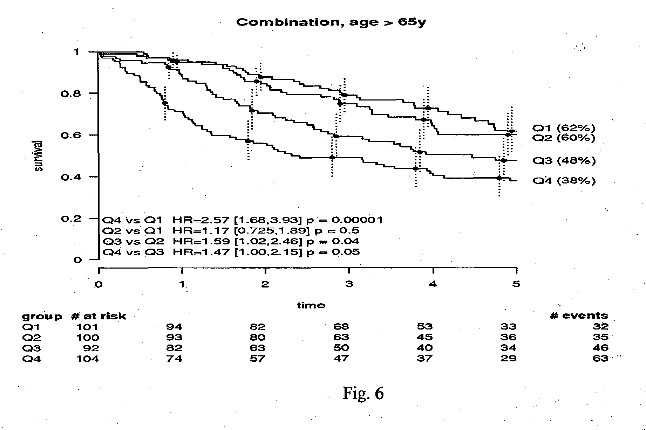

Each of the signatures (module 1,2, and 3) and the combination are shown separately in Fig 3A-F, Fig 4A-F, Fig 5A-F and Fig 6A-F, respectively. Specifically, Fig. 3A-F shows the prognostic performance of module 1 under stratification by stage, histology and age at diagnosis. The quartiles are the same as in Figure 2 (that is, the grouping is made first, before the startification). Here, as shown, the prognostic power remains within each strata. Fig. 4A-F shows the prognostic performance of module 2 under stratification by stage, histology and age at diagnosis. Fig. 5A-F shows the prognostic performance of module 3 under stratification by stage, histology and age at diagnosis. Fig. 6A-F shows the prognostic performance of the combined score of module 1, 2, and 3 under stratification by stage, histology and age at diagnosis.

In most instances, the prognostic power of the signatures (individually and in combination) was still observed. This indicates that the proposed signatures provided additional prognosis beyond the traditional factors. That is, the signatures are not merely surrogate markers that are highly correlated with existing factors. In particular, patients with the same tumor stage can be distinguished further into a range of risks. This was not merely refinement of the staging system, since the risk ordering may be reversed. For example, the group of patients with the highest risk in stage I is actually having worse survival than the average survival of stage II patients.

The analyses under various contexts of existing clinico-pathologic factors also highlight that these factors can also be incorporated in the application of the signatures. For example, for squamous cell carcinoma, the individual signatures did not show substantial risk discrimination, but the combined score shows the top quartile having substantially a worst outcome than the rest.

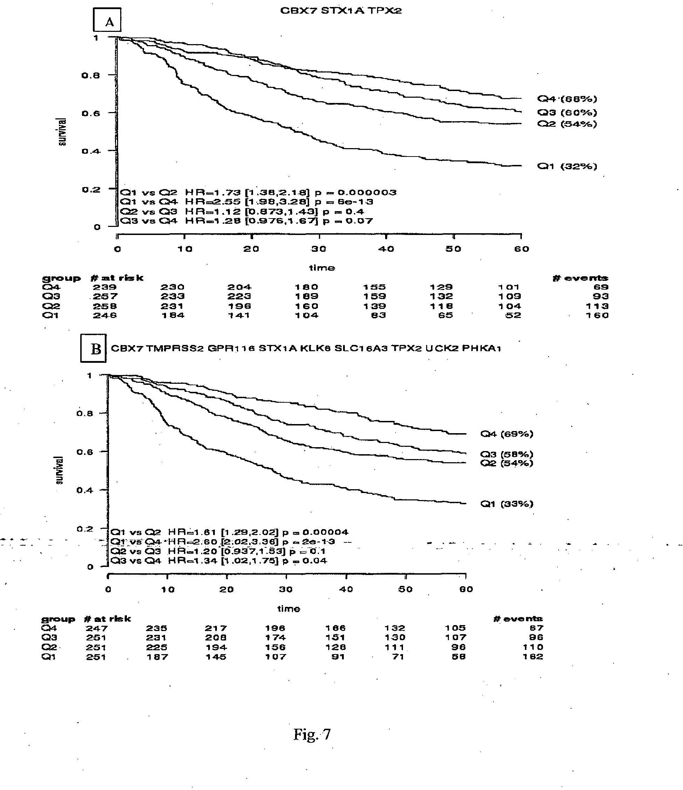

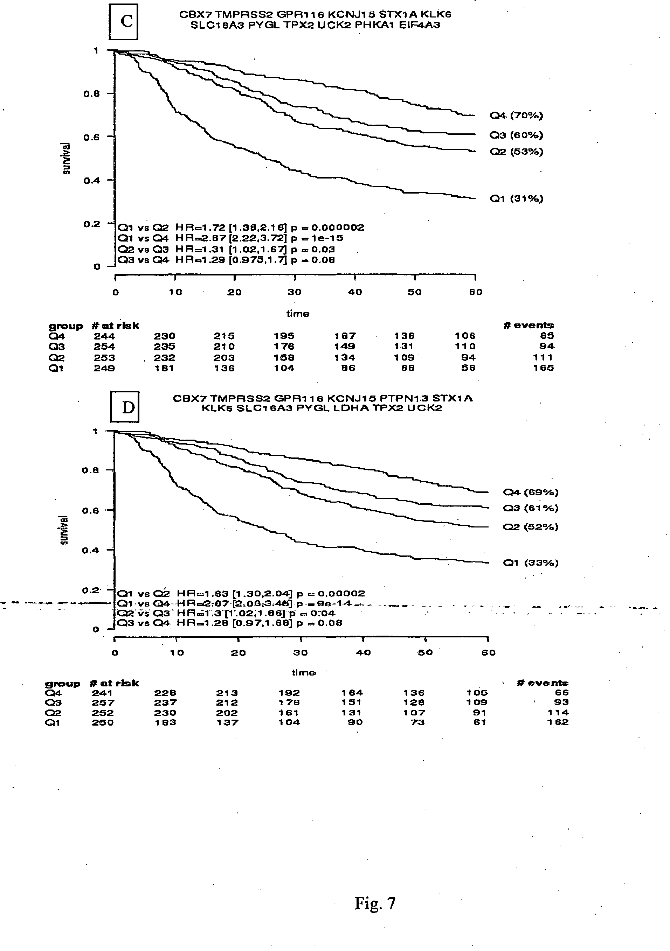

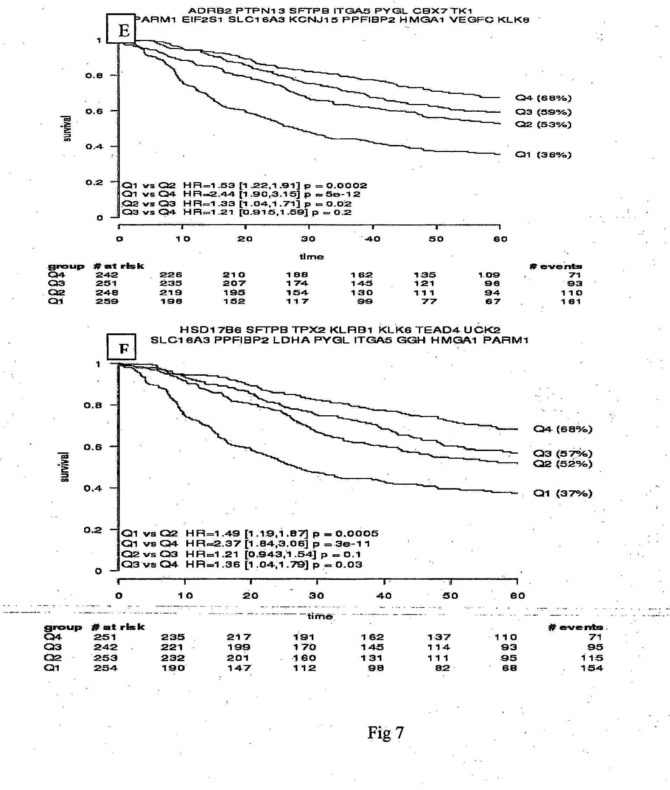

Figure 7A-F shows the prognostic performance of several example variations of the claimed signature obtained by selecting subsets of genes from Table 1, 2, and/or 3. The ability to separate the patients according to survival is not comprised by not having the complete set of genes. A minimal signature made of three genes (one from each table 1,2 and 3) already performs quite well (panel A). Separations between groups are improved as the number of genes are increased (panel B-D). Arbitrarily chosen 15 genes perform well (panel E and F).

### Individualized Therapy Decision Making

The risk prediction system will utilize database of clinical and gene expression data, similar to the one used in this example to allow projection of risk under alternative treatments (including no treatment). This system is similar to AdjuvantOnline (Ravdin et al 2001, J. Clin. Oncol. 19:980), except that it also includes the scores from the claimed invention.

### Example 2:

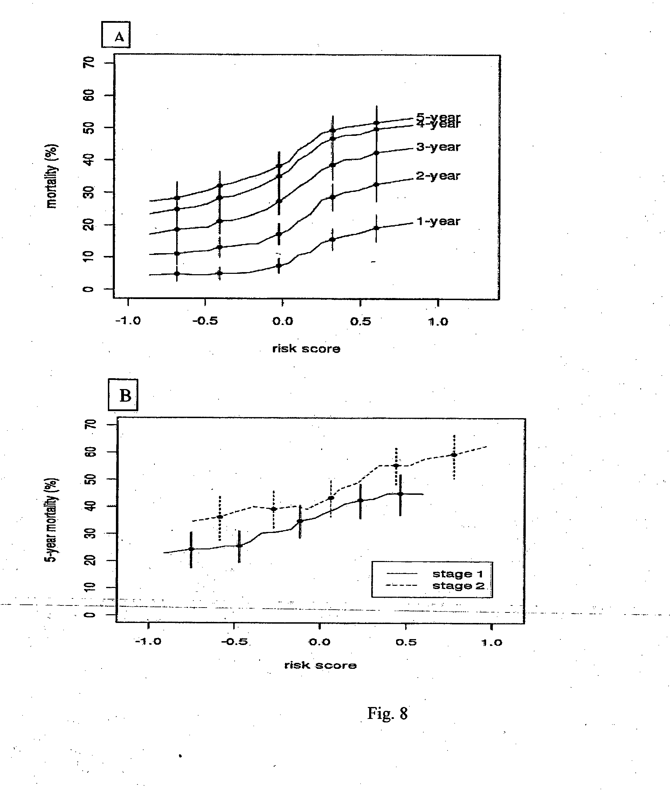

Typical application of the biomarkers disclosed in Tables 1, 2 and/or 3 for a patient with lung cancer.
1. A patient diagnosed with lung cancer with small and operable primary tumors undergoes surgery to remove the lession. A part of the tumor tissue is examined by standard pathology procedure such as determination of tumor size, tumor histological types (such as adenocarcinoma, squamous cell carcinoma, or other types). Tumor staging is determined using standard guidelines, based on tumor size, presence of lymph node metastasis or other distant sites of metastasis. The information obtained from the standard clinico-pathologic measurements may modify and enhance the prognostic and predictive application of the invention, but it is not a requirement and not an integral part.
2. A part of the tumor tissue is used as the source material for the claimed invention, either as frozen, paraffin embedded or fresh tissue. Whole transcriptome RNA extraction is performed on the tissue.
3. Measurement of the relative quantity of the RNA for specific set of genes (claimed in the invention) is performed by any of these procedures:
   - qNPA technology or nanoString technology
   - Quantitative RT-PCR of specific genes
   - Hybridization to microarrays containing either selected probes or whole transcriptome
   - High-throughput sequencing of the RNA (RNA-seq), followed by computational selection and quantitation of the relevant genes
4. The relative abundance of the RNA for each is calibrated either by a computational normalization procedure against a set of control genes that are expected to have constant expression across all patients (Popovici et al. (2009) Selecting control genes for RT-QPCR using public microarray data. BMC Bioinformatics. 2009 Feb 2;10:42), or by normalization against a database of similar measuremnts from existing lung tumors (McCall et al. (2010) Biostatistics. 2010 Apr;11 (2):242-53. Epub 2010 Jan 22.)
5. The relative abundance is log transformed, and weighted averages from multiple genes in a set is computed. The weights can be as simple as +1 or -1 (positive or negative sign, depending on the effect of the change of gene expression on the outcome) (Sotiriou et al. (2006) J Natl Cancer Inst. 2006 Feb 15;98(4):262-72.) or they can be numbers calibrated to the specific measurement techniques. These weighted averages is considered to be the raw risk scores to be used as input in subsequent risk calculation. Each signature module has its own risk score.
6. The risk profile calculator provides a projection/prediction of the probability either disease-free state, metastasis-free state or survival of an individual patient, for any time point in the future (measured in years after surgery). The reliability of the projection is characterized by time-specific confidence interval that depends on the quantity and quality of the reference database. Near-term projection is typically supported by more data and therefore more reliable (as indicated by narrower confidence interval) compared to long-term prediction. An example of risk profile calculator chart is shown in Fig. 8A, where a measured risk score for a specific patient is translated to lung cancer mortality (based on a reference database, using nearest-neighbor Kaplan-Meier estimates).
7. The risk projection can be modulated by information other than the risk scores provided by the invention, such as the age and gender of the patients, Karnofsky performance score, tumor size and lymph node status. Integration of all these factors into a specific risk profile for a given patient may be done using well-established survival regression method (Therneau TM, Grambsch PM (2000) Modelling survival data: extending the Cox model. Springer, New York), such as
   - parametric regression models (e.g. using exponential or Weibull models)
   - semi-parametric method (Cox regression)
   - empirical survival curve estimators such as Kaplan-Meier or actuarial methods for each possible combination of factors

An example of how the risk prediction is modulated by tumor stage is shown in Fig. 8B. Stage 2 patients show higher 5-year mortality than stage 1 patients, as expected. However, a subset of stage 1 patient that has high risk score (higher than zero, in standardized risk score unit), show mortality rate very similar to that of the average stage 2 patients (dashed curve). This indicates that the adjuvant chemotherapy by staging alone might be inadequate because some patients in stage 1 (untreated under current guideline) are in fact has similar risk to many stage 2 patients (treated under current guideline).

The risk projection can be calibrated against a database of past observations of patients with records of disease outcome, clinico-pathologic variables and measurements of the claimed invention. The database may be periodically updated with information from new patients. This system is similar to AdjuvantOnline (Ravdin et al. (2001) J Clin Oncol. 2001 Feb 15;19(4):980-91), a widely used tool for projecting the survival of cancer patients as the function of various clinico-pathologic variables.

We extend the system to include multi-modal scores derived from genomics and transcriptomics technology.

8. The modulating factors put into the risk calculator may include commonly used adjuvant therapy (such as platinum-based chemotherapy) or anti-angiogenesis drugs. In this scenario of response-prediction applications, two or more risk profiles will be presented, corresponding to the projected probability of outcome under alternative treatments, or no treatment.

### Example 3

Application of the signature to formalin-fixed paraffin embedded (FFPE) tumor materials.

The claimed signatures (the set of genes and the formula for deriving the risk scores) can be directly applied to expression data from technology platforms such as Affymetrix, qNPA or nanoString, after tissue preparation and raw data preprocessing procedure suitable for each respective platform.

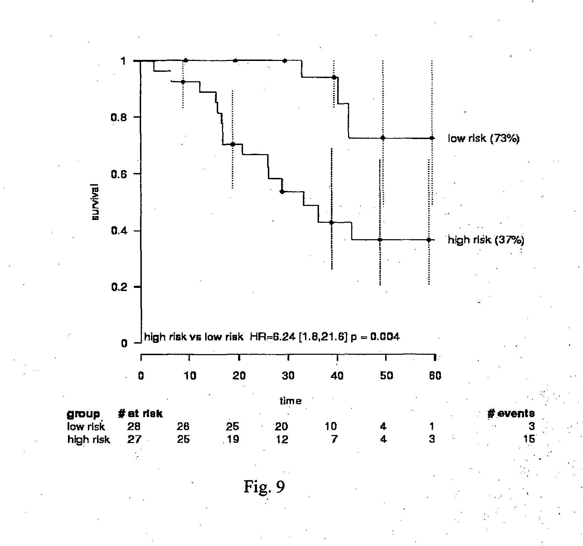

Fig. 9 shows the survival performance in publicly available dataset from M. D. Anderson Cancer Center (Xie et al. 2011 Clin Cancer Res 17:5705-14; Gene Expression Omnibus accession GSE29013) which were obtained from FFPE material. Here, significant prognostic power is still observed, despite the generally lower quality (larger noise level, larger number of absent calls of expression value) in Affymetrix data from FFPE.

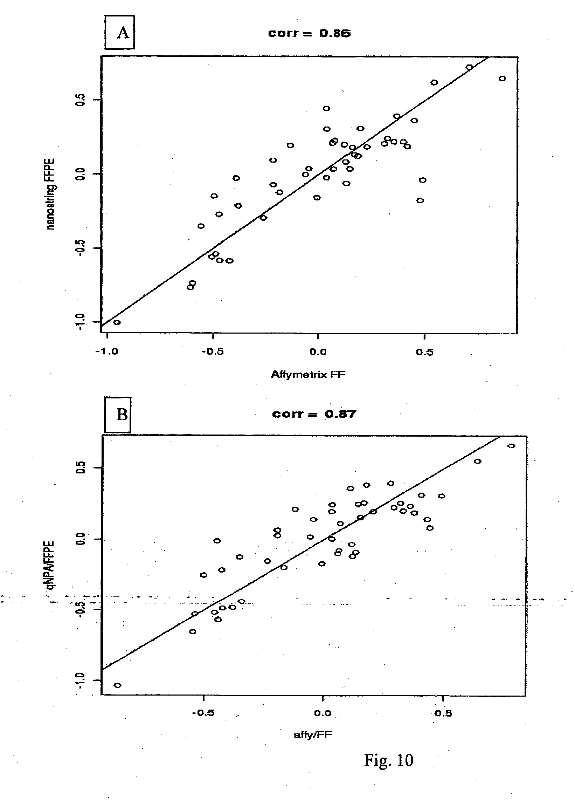

To assess whether the signature risk scores can potentially provide the same prognostic value in qNPA and nanoString data with FFPE as in Affymetrix with fresh frozen tissue, comparisons were performed on materials from the same lung cancer patients (unpublished data). Fig.10A-B show the pairwise scatter plots comparing FF/Affymetrix vs FFPE/qNPA, as well as FF/Affymetrix vs FFPE/nanoString. High correlations (0.86 and 0.87 respectively) were observed, showing that the claimed signature is not dependent on the choice of technology platforms, nor the tissue preservation methods.

## Claims

1. A method for prognosing a subject with non-small cell lung cancer (NSCLC) comprising:
using a test sample obtained from a subject suffering from NSCLC following surgical resection;
determining the expression level of a combination of biomarkers, wherein the combination of biomarkers comprise CBX7, TMPRSS2, GPR116, STX1A, KLK6, SLC16A3, TPX2, UCK2, and PHKA1; and
analyzing the expression level to generate a risk score, wherein the risk score can be used to provide a prognosis of the subject.

2. A method for prognosing a subject with non-small cell lung cancer (NSCLC) comprising:
using a test sample obtained from a subject suffering from NSCLC following surgical resection;
determining the expression level of a combination of biomarkers wherein the combination of biomarkers comprise CBX7, STX1A, and TPX2; and
analyzing the expression level to generate a risk score, wherein the risk score can be used to provide a prognosis of the subject.

3. The method of claim 1 or claim 2 wherein the risk score classifies the subject in a high risk group and would benefit from receiving adjuvant chemotherapy or in a low risk group and would not benefit from receiving adjuvant chemotherapy.

4. The method of claim 1 or claim 2, wherein the subject has a prognosis of having poor survival or a prognosis of having good survival.

5. The method of claim 2, wherein the combination of biomarkers further comprise, TMPRSS2, KLK6, and UCK2.

6. The method of claim 2, wherein the combination of biomarkers further comprise TMPRSS2, GPR116, , KLK6, SLC16A3, UCK2, and PHKA1.

7. The method of claim 1, wherein the combination of biomarkers further compriseKCNJ15, , PYGL, and EIF4A3.

8. The method of claim 1, wherein the combination of biomarkers further comprise KCNJ15, PTPN13,, PYGL, LDHA, EIF4A3 and TK1.

9. The method of claim 1, wherein the combination of biomarkers further comprise KCNJ15, PTPN13, CTSH, , PYGL, LDHA, ITGA5, EIF4A3, TK1, and CCNA2.

10. The method of any one of the preceding claims, wherein the test sample is fresh, frozen, parrafin fixed embedded cells.

11. The method of any one of the preceding claims, wherein the expression level is determined using qNPA, nanoString, quantitative PCR or an array.

12. The method of claim 1 or claim 2, wherein analyzing expression to generate a risk score is performed using statistical analysis.

13. The method of claim 12, wherein the statistical analysis comprises Cox regression analysis or parametric survival predictors. ,

14. The method of the preceding claims, wherein the subject is a human.

## Patentansprüche

1. Verfahren zur Prognose eines Probanden, der an nichtkleinzelligem Bronchialkarzinom (NSCLC) leidet, umfassend:
Verwendung einer Testprobe, die nach chirurgischer Resektion von einem Probanden erhalten wird, der an NSCLC leidet;
Feststellen des Expressionspegels einer Kombination aus Biomarkern, wobei die Kombination aus Biomarkern CBX7, TMPRSS2, GPR116, STX1A, KLK6, SLC16A3, TPX2, UCK2 und PHKA1 umfasst; und
Analysieren des Expressionspegels, um eine Risikoauswertung zu generieren, wobei die Risikoauswertung verwendet werden kann, um eine Prognose des Probanden bereitzustellen.

2. Verfahren zur Prognose eines Probanden, der an nichtkleinzelligem Bronchialkarzinom (NSCLC) leidet, umfassend:
Verwendung einer Testprobe, die nach chirurgischer Resektion von einem Probanden erhalten wird, der an NSCLC leidet;
Feststellen des Expressionspegels einer Kombination aus Biomarkern, wobei die Kombination aus Biomarkern CBX7, STX1A und TPX2 umfasst; und
Analysieren des Expressionspegels, um eine Risikoauswertung zu generieren, wobei die Risikoauswertung verwendet werden kann, um eine Prognose des Probanden bereitzustellen.

3. Verfahren nach Anspruch 1 oder Anspruch 2, wobei die Risikoauswertung den Probanden in eine hohe Risikogruppe einstuft und dieser von einer unterstützenden Chemotherapie Nutzen tragen könnte, oder in eine niedrige Risikogruppe einstuft und keinen Nutzen von einer unterstützenden Chemotherapie tragen würde.

4. Verfahren nach Anspruch 1 oder Anspruch 2, wobei der Proband eine Prognose einer schlechten Überlebenschance oder eine Prognose einer guten Überlebenschance aufweist.

5. Verfahren nach Anspruch 2, wobei die Kombination aus Biomarkern ferner TMPRSS2, KLK6 und UCK2 umfasst.

6. Verfahren nach Anspruch 2, wobei die Kombination aus Biomarkern ferner TMPRSS2, GPR116, KLK6, SLC16A3, UCK2 und PHKA1 umfasst.

7. Verfahren nach Anspruch 1, wobei die Kombination aus Biomarkern ferner KCNJ15, PYGL und EIF4A3 umfasst.

8. Verfahren nach Anspruch 1, wobei die Kombination aus Biomarkern ferner KCNJ15, PTPN13, PYGL, LDHA, EIF4A3 und TK1 umfasst.

9. Verfahren nach Anspruch 1, wobei die Kombination aus Biomarkern ferner KCNJ15, PTPN13, CTSH, PYGL, LDHA, ITGA5, EIF4A3, TK1 und CCNA2 umfasst.

10. Verfahren nach einem der vorstehenden Ansprüche, wobei es sich bei der Testprobe um frische, gefrorene, paraffinfixierte, eingebettete Zellen handelt.

11. Verfahren nach einem der vorstehenden Ansprüche, wobei der Expressionspegel unter Verwendung von qNPA, nanoString, quantitativer PCR oder einer Anordnung festgestellt wird.

12. Verfahren nach Anspruch 1 oder Anspruch 2, wobei das Analysieren der Expression zum Generieren einer Risikoauswertung unter Verwendung einer statistischen Analyse durchgeführt wird.

13. Verfahren nach Anspruch 12, wobei die statistische Analyse eine Cox-Regressionsanalyse oder parametrische Überlebensvoraussagen umfasst.

14. Verfahren nach den vorstehenden Ansprüchen, wobei es sich beim Probanden um einen Menschen handelt.

## Revendications

1. Procédé de détermination d'un pronostic chez un patient atteint du cancer du poumon non à petites cellules (CPNPC) comprenant :
l'utilisation d'un échantillon à tester prélevé sur un patient souffrant de CPNPC suite à une résection chirurgicale ;
la détermination du niveau d'expression d'une association de biomarqueurs, dans lequel l'association de biomarqueurs comprend CBX7, TMPRSS2, GPR116, STX1A, KLK6, SLC16A3, TPX2, UCK2 et PHKA1 ; et
l'analyse du niveau d'expression pour générer un score de risque, dans lequel le score de risque peut être utilisé pour déterminer un pronostic chez le patient.

2. Procédé de détermination d'un pronostic chez un patient atteint du cancer bronchopulmonaire non à petites cellules (CBNPC) comprenant :
l'utilisation d'un échantillon à tester prélevé sur un patient souffrant de CBNPC suite à une résection chirurgicale ;
la détermination du niveau d'expression d'une association de biomarqueurs, dans lequel l'association de biomarqueurs comprend CBX7, STX1A et TPX2 ; et
l'analyse du niveau d'expression pour générer un score de risque, dans lequel le score de risque peut être utilisé pour déterminer un pronostic chez le patient.

3. Procédé selon la revendication 1 ou la revendication 2, dans lequel le score de risque permet de classer le patient dans un groupe à haut risque et qui bénéficierait de l'administration d'une chimiothérapie adjuvante ou dans un groupe à faible risque et qui ne bénéficierait pas de l'administration d'une chimiothérapie adjuvante.

4. Procédé selon la revendication 1 ou la revendication 2, dans lequel le patient a un pronostic de survie médiocre ou un bon pronostic de survie.

5. Procédé selon la revendication 2, dans lequel l'association de biomarqueurs comprend en outre TMPRSS2, KLK6 et UCK2.

6. Procédé selon la revendication 2, dans lequel l'association de biomarqueurs comprend en outre TMPRSS2, GPR116, KLK6, SLC16A3, UCK2 et PHKA1.

7. Procédé selon la revendication 1, dans lequel l'association de biomarqueurs comprend en outre KCNJ15, PYGL et EIF4A3.

8. Procédé selon la revendication 1, dans lequel l'association de biomarqueurs comprend en outre KCNJ15, PTPN13, PYGL, LDHA, EIF4A3 et TK1.

9. Procédé selon la revendication 1, dans lequel l'association de biomarqueurs comprend en outre KCNJ15, PTPN13, CTSH, PYGL, LDHA, ITGA5, EIF4A3, TK1 et CCNA2.

10. Procédé selon l'une quelconque des revendications précédentes, dans lequel l'échantillon à tester est un échantillon de cellules fraîches, congelées, enrobées et fixées dans de la paraffine.

11. Procédé selon l'une quelconque des revendications précédentes, dans lequel le niveau d'expression est déterminé en utilisant une technique de dosage qNPA, une plateforme NanoString, une PCR quantitative ou une puce à ADN.

12. Procédé selon la revendication 1 ou la revendication 2, dans lequel l'analyse de l'expression pour générer un score de risque est effectuée en utilisant une analyse statistique.

13. Procédé selon la revendication 12, dans lequel l'analyse statistique comprend l'analyse de régression de Cox ou les prédicteurs de survie paramétriques.

14. Procédé selon l'une quelconque des revendications précédentes, dans lequel le patient est un être humain.
